(19) Europäisches Patentamt
European Patent Office
Office européen des brevets

(11) Numéro de publication: **0 628 562 A1**

# (12) DEMANDE DE BREVET EUROPEEN

(21) Numéro de dépôt: **93402971.1**

(51) Int. Cl.$^5$: **C07D 501/20**, A61K 31/545

(22) Date de dépôt: **09.12.93**

(30) Priorité: **10.06.93 FR 9306975**

(43) Date de publication de la demande: **14.12.94 Bulletin 94/50**

(84) Etats contractants désignés: **AT BE CH DE DK ES FR GB GR IE IT LI LU NL PT SE**

(71) Demandeur: **ROUSSEL-UCLAF**
**35, Boulevard des Invalides**
**F-75007 Paris (FR)**

(72) Inventeur: **Aszodi, Jozsef**
**90, rue Robespierre**
**F-77340 Pontault Combault (FR)**
Inventeur: **Dini, Christophe**
**6, Allée des Tamaris**
**F-91220 Le Plessis Pate (FR)**
Inventeur: **Fauveau, Patrick**
**40, Avenue Camille Desmoulins**
**F-93190 Livry Gargan (FR)**

(74) Mandataire: **Vieillefosse, Jean-Claude et al**
**ROUSSEL UCLAF,**
**111 Route de Noisy**
**F-93235 Romainville Cédex (FR)**

## (54) Céphalosporines comportant en position 7 un radical oxymino substitué, leurs intermédiaires, leurs procédé de préparation et leur application comme médicaments.

(57) L'invention a pour objet les produits de formule générale (I) :

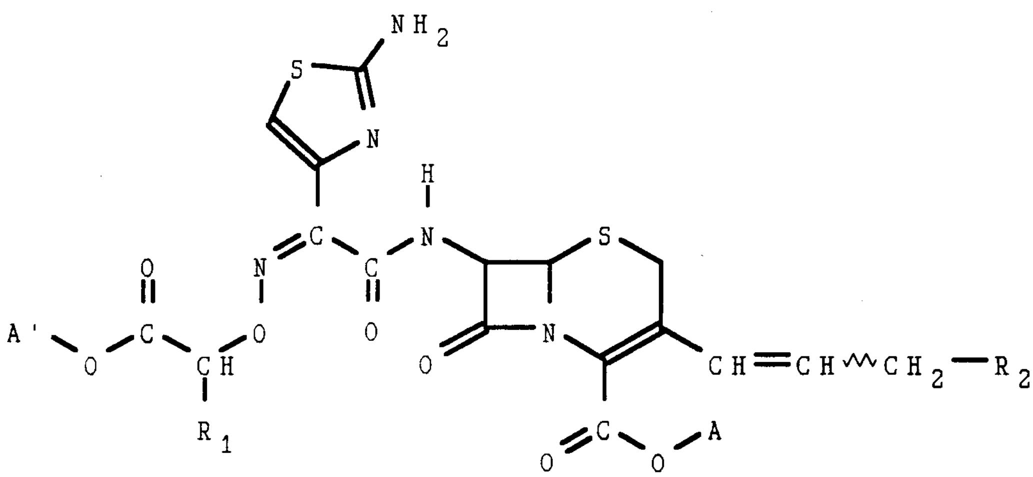

(I)

isomère syn, sous forme (R) ou (S) ou d'un mélange (R,S), sous forme de sel interne ou de leurs sels avec les acides minéraux organiques, formule dans laquelle :
$R_1$ représente un groupement de formule (K) :

EP 0 628 562 A1

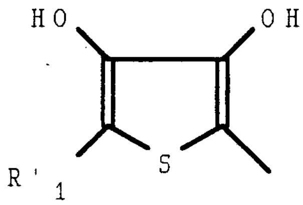

(K)

dans laquelle $R'_1$ est alkyle (1 à 4 carbones), cyano, carboxy ou alkoxycarbonyle, (alkyle 1 à 4 carbones), ou $R_1$ représente un groupement de formule (L) :

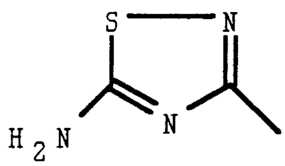

(L)

A et A', représentent un hydrogène, un équivalent de métal alcalin, alcalinoterreux, de magnésium, d'ammonium ou d'une base organique aminée, ou bien un seul ou chacun des groupes $CO_2A$ ou $CO_2A'$ représentent $CO_2^{\ominus}$ et $R_2$ un ammonium quaternaire.

Ces produits possèdent d'intéressantes propriétés pharmacologiques qui justifient leur emploi comme médicaments.

La présente invention concerne de nouvelles céphalosporines comportant sur la chaîne latérale en position 7, un radical oxyimino substitué, leur procédé de préparation, leur application comme médicaments, les compositions les renfermant et les nouveaux intermédiaires obtenus.

L'invention a pour objet les produits de formule générale (I) :

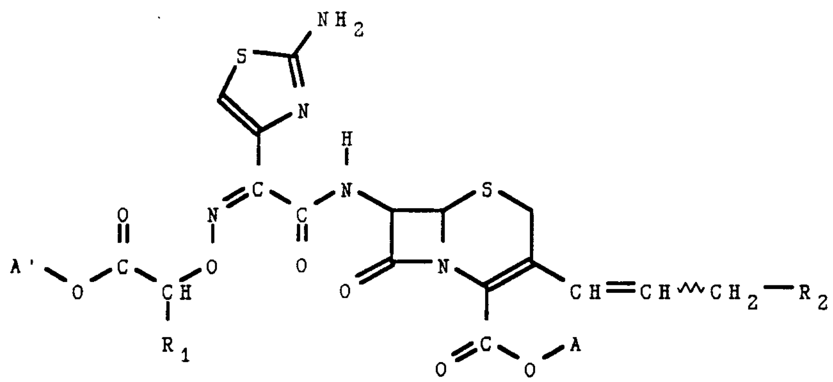

(I)

isomère syn, sous forme (R) ou (S) ou d'un mélange (R,S), sous forme de sel interne ou de leurs sels avec les acides minéraux organiques, formule dans laquelle :

$R_1$ représente un groupement de formule (K) :

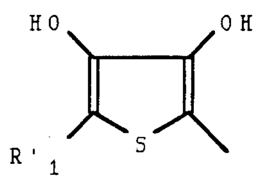

(K)

dans laquelle $R'_1$ représente un radical alkyle renfermant de 1 à 4 atomes de carbone, un radical cyano, carboxy ou alkoxycarbonyle, dans lequel le radical alkyle renferme de 1 à 4 atomes de carbone, ou $R_1$ représente un groupement de formule (L) :

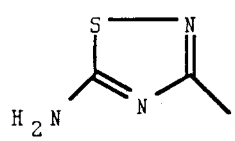

(L)

A et A', identiques ou différents, représentent un atome d'hydrogène, un équivalent de métal alcalin, alcalinoterreux, de magnésium, d'ammonium ou d'une base organique aminée,

ou bien un seul ou chacun des groupes $CO_2A$ ou $CO_2A'$ représentent $CO_2^{\ominus}$,

le trait ondulé signifie que le groupement $CH_2R_2$ peut se trouver dans la position E ou Z et $R_2$ représente sous forme d'ammonium quaternaire, un des radicaux suivants :

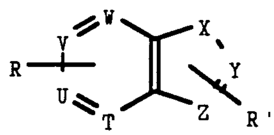
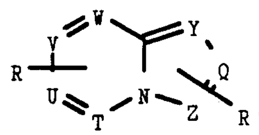
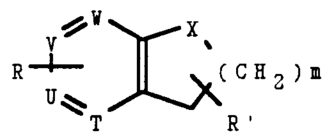
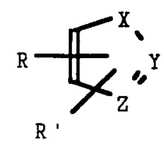

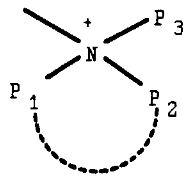

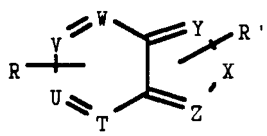
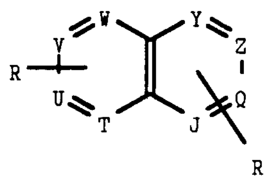
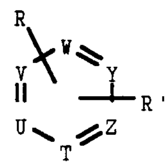
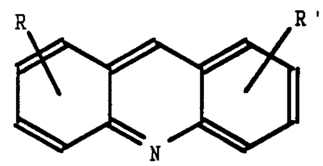

dans lesquels X représente $CH_2$, NH, O ou S ;

Q, J, Y, T, U, V, W et Z, identiques ou différents, représentent indépendamment les uns des autres CH ou N, étant entendu que chacun de ces radicaux cycliques contient de 1 à 5 hétéroatomes, qu'au moins un de ces hétéroatomes est l'atome d'azote et que ces radicaux cycliques sont substitués par un ou plusieurs radicaux R ou R' ;

R et R', identiques ou différents, représentent un atome d'hydrogène, un atome d'halogène, un radical alkyle renfermant de 1 à 4 atomes de carbone, un radical alkyloxy renfermant de 1 à 4 atomes de carbone, un atome d'halogène, un radical cyano, un radical $CO_2$-$Q_1$, CO-$NQ_1(Q_2)$, $NQ_1(Q_2)$, $SO_2$-$NQ_1(Q_2)$, CS-$NH_2$, NH-CO-$Q_1$, CH=N-OH, CH=N-O-$Q_1$, $CH_2$-CN, $CH_2$-S-$Q_1$ dans lesquels $Q_1$ et $Q_2$, identiques ou différents, représentent un atome d'hydrogène ou un radical alkyle renfermant de 1 à 4 atomes de carbone, $P_1$, $P_2$ et $P_3$, identiques ou différents, représentent un radical alkyle renfermant de 1 à 4 atomes de carbone, éventuellement substitué par un des substituants ayant les définitions indiquées ci-dessus pour R et R', le trait pointillé indique que $P_1$ et $P_2$ peuvent éventuellement former avec l'atome d'azote auquel ils sont liés, un hétérocycle à 5 ou 6 chaînons.

Par radical alkyle renfermant de 1 à 4 atomes de carbone, on entend les radicaux, méthyle, éthyle, propyle, isopropyle, butyle, isobutyle, sec-butyle ou tert-butyle.

Par radical alkyloxy renfermant de 1 à 4 atomes de carbone, on entend les radicaux, méthoxy, éthoxy, propoxy, isopropoxy, butoxy, isobutoxy, sec-butoxy ou tert-butoxy.

Par atome d'halogène, on entend atome de fluor, chlore, brome ou iode.

Lorsque $P_1$ et $P_2$ forment un hétérocycle avec l'atome d'azote auquel ils sont liés, il peut s'agir d'une pyrrolidine, d'une morpholine ou d'une pipéridine.

Les produits de formule (I) peuvent exister sous forme de sels. Parmi les valeurs de A et A', on peut citer un équivalent de sodium, de potassium, de lithium, de calcium, de magnésium ou d'ammonium. On peut citer, parmi les bases organiques, la méthylamine, la propylamine, la triméthylamine, la diéthylamine, la triéthylamine, la N,N-diméthyléthanolamine, le tris[(hydroxyméthyl) amino] méthane, l'éthanolamine, la pyridine, la picoline, la dicyclohexylamine, la morpholine, la benzylamine, la procaïne, la lysine, l'arginine, l'histidine, la N-méthylglucamine.

Les produits de formule (I) peuvent également se présenter sous forme de sel interne pur, sous forme salifiée ou sous forme associée aux acides de la solution. Parmi les acides avec lesquels on peut salifier le ou les produits de formule (I), on peut citer entre autres, les acides acétique, trifluoroacétique, maléïque, tartrique, méthanesulfonique, benzènesulfonique, para-toluènesulfonique, phosphorique, sulfurique, chlorhydrique, bromhydrique, iodhydrique.

Dans un mode préféré de l'invention, A' représente un atome d'hydrogène et $CO_2A$ représente $CO_2^{\ominus}$.

L'expression sous forme d'ammonium quaternaire indique que le radical $R_2$ est lié par le ou l'un des atomes d'azote qu'il comporte.

L'invention a particulièrement pour objet les produits de formule générale (I) telle que définie ci-dessus dans laquelle $R_2$ représente un des radicaux suivants :

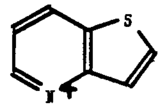
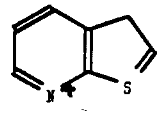
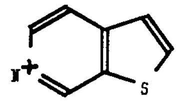
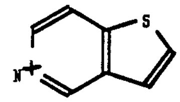
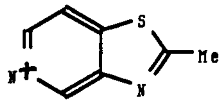
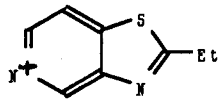
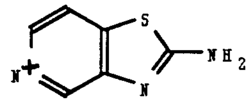
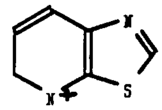
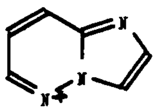
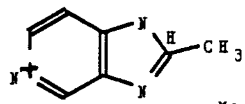
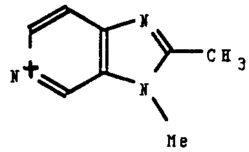
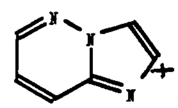
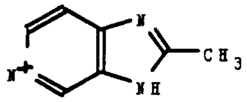
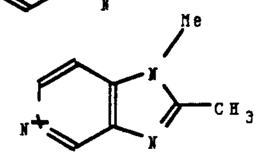
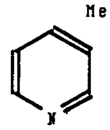
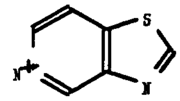
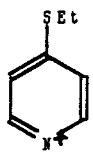
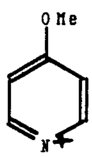
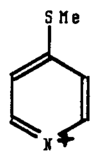
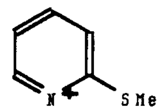
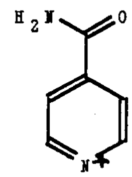
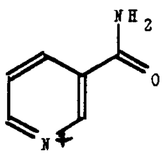
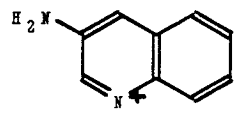
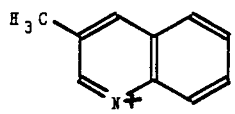
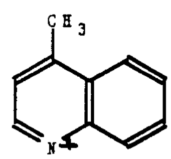

ou encore l'un des radicaux suivants :

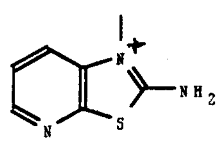
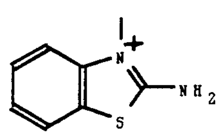
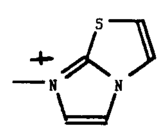
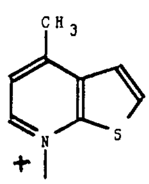
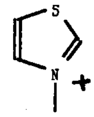

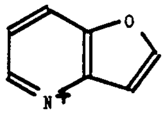 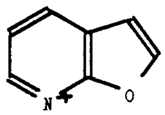 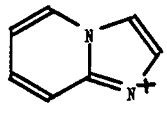 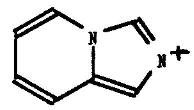

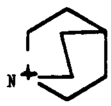 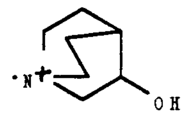 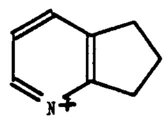 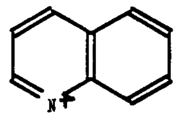

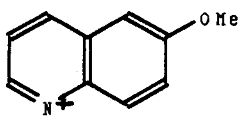 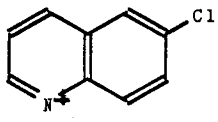 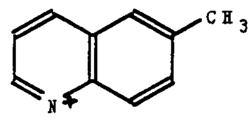

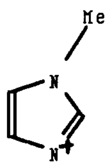 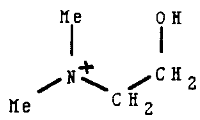 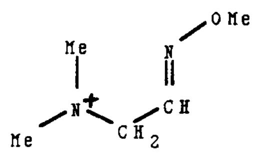 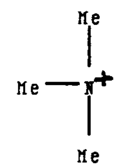

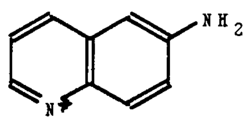 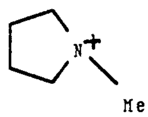 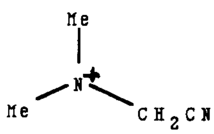 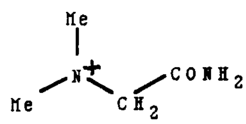

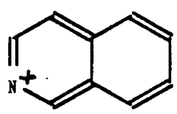 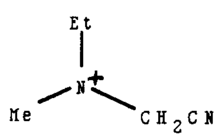 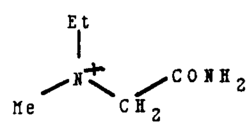

L'invention a plus particulièrement pour objet les produits de formule générale (I) telle que définie ci-dessus dans laquelle $R_2$ représente le radical quinoléinium, isoquinoléinium, 4-(méthylthio) pyridinium, thièno[2,3-b]pyridinium, imidazo(1,2-a)pyridinium ou le radical 6,7-dihydro-5H-pyrindinium, ceux dans laquelle $R_1$ représente un radical de formule (K) dans laquelle $R'_1$ représente un radical cyano, carboxy ou alkoxycarbonyle.

L'invention a tout particulièrement pour objet le produit dont le nom suit :

- le sel interne de [6R-[3-(E) 6alpha, 7béta-(Z)]] 1-[3-[7-[[(2-amino 4-thiazolyl) [[carboxy 5-cyano 3,4-dihydroxy 2-thiényl) méthoxy] imino] acétyl] amino] 2-carboxy 8-oxo 5-thia 1-azabicyclo[4,2,0]oct-2-èn-3-yl] 2-propényl] quinoléinium.

L'invention a également pour objet un procédé de préparation des produits de formule (I) telle que définie ci-dessus, caractérisé en ce que l'on traite un ester de formule (II) :

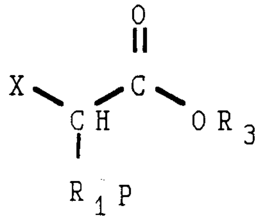 (II)

dans laquelle X représente un groupement partant ou un radical susceptible de générer in situ un groupement partant, $R_1p$ représente un groupement $R_1$ tel que défini plus haut, dans lequel les radicaux

hydroxy ou amino sont protégés et $R_3$ représente le reste d'un ester aisément clivable, par le N-hydroxy phtalimide, le cas échéant en présence d'un agent activant, pour conduire au dérivé de formule (III) :

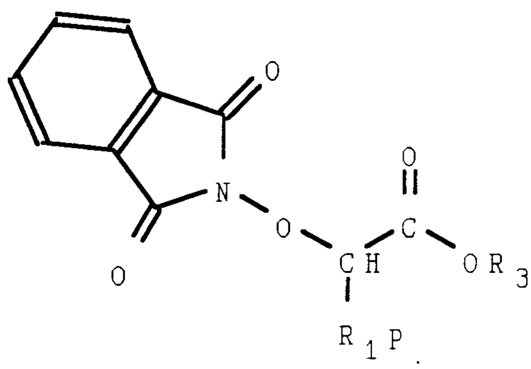

(III)

dérivé de formule (III) que l'on hydrolyse en hydroxylamine O-substituée de formule (IV) :

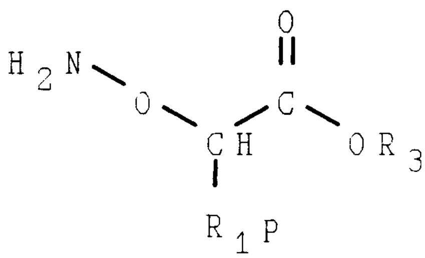

(IV)

produit de formule (IV) que l'on condense avec un dérivé de l'acide (2-amino thiazol-4-yl) 2-oxoacétique de formule (V) :

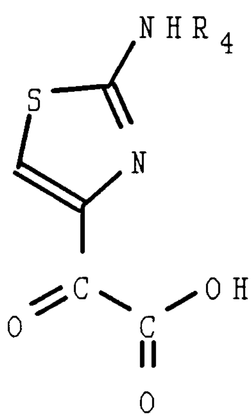

(V)

dans laquelle $R_4$ représente un atome d'hydrogène ou un groupe protecteur de la fonction amine, pour former le dérivé de l'acide alpha-alkoxy imino acétique de formule (VI) :

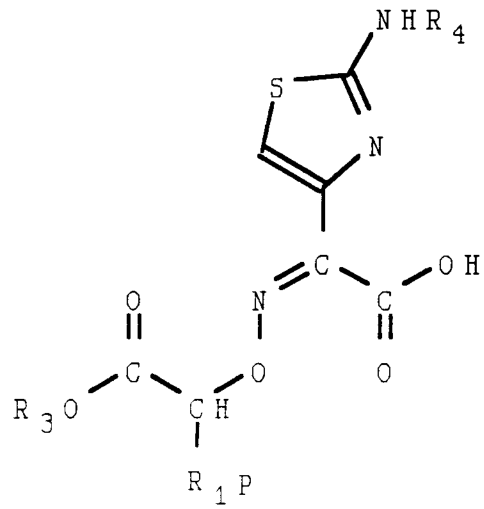

**(VI)**

dont on prépare, le cas échéant, un dérivé fonctionnel, produit de formule (VI) ou dérivé fonctionnel que l'on amidifie avec un ester du chlorhydrate de l'acide 7-amino 3-(3-halogéno 1-propènyl) 8-oxo 5-thia 1-azabicyclo[4,2,0]oct-2-èn-2-carboxylique de formule (VII) :

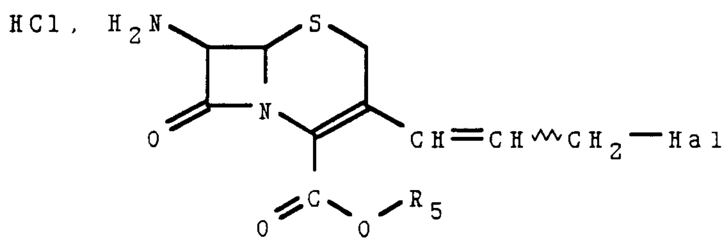

**(VII)**

ou de ses sels, dans laquelle Hal représente un atome d'halogène et $R_5$ représente le reste d'un ester aisément clivable, pour conduire au dérivé de l'acide 7-(N-substitué amido) 3-(3-halo 1-propènyl) 8-oxo 5-thia 1-azabicyclo[4,2,0]oct-2-èn-2-carboxylique de formule (VIII) :

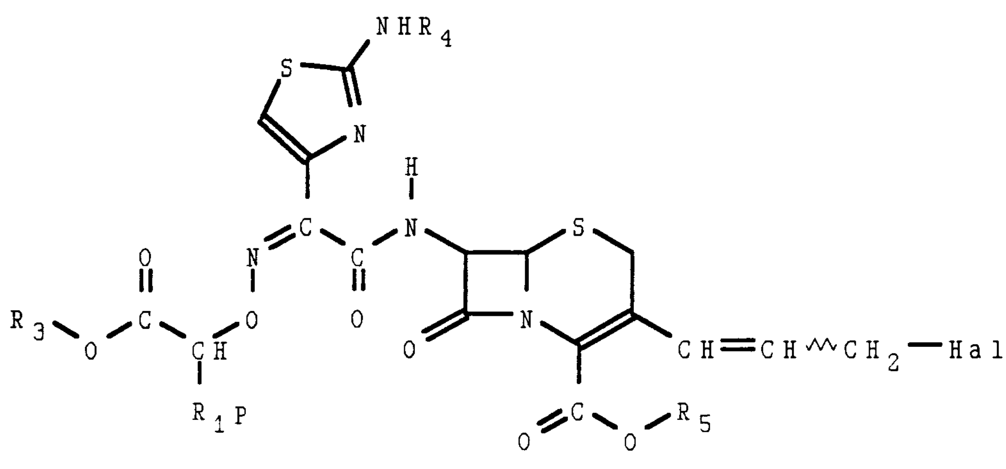

**(VIII)**

produit de formule (VIII) que l'on transforme, le cas échéant, en analogue 3-(3-iodo propènylé) de formule (IX) :

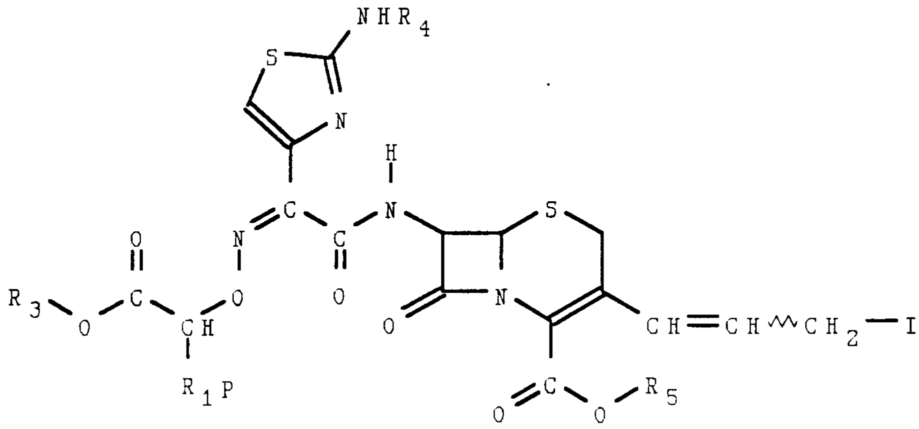

(IX)

produit de formule (IX) que l'on traite avec une base de formule $R_2$ pour obtenir le produit de formule (X) :

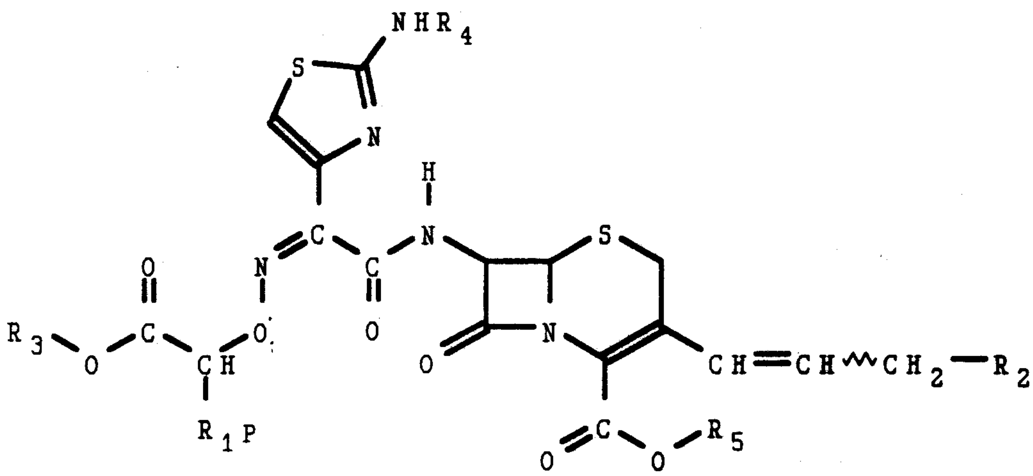

(X)

produit de formule (X) à partir duquel si désiré, l'on isole les isomères (E) ou (Z) ou transforme les isomères (Z) en isomère (E) et produit de formule (X) que l'on soumet à une ou plusieurs des réactions suivantes, dans un ordre quelconque :

a) coupure par hydrolyse ou par action de la thiourée de tout ou partie des groupements esters ou des groupements de protection du ou des radicaux amino ou des radicaux hydroxyles,

b) estérification ou salification par une base du ou des radicaux carboxyliques,

c) salification par un acide du ou des radicaux amino,

d) séparation des produits sous forme de mélange R,S en R ou S.

L'invention a aussi pour objet une variante du procédé décrit ci-dessus, caractérisé en ce que l'hydroxylamine O-substituée de formule (IV) est condensée au produit de formule (XI) :

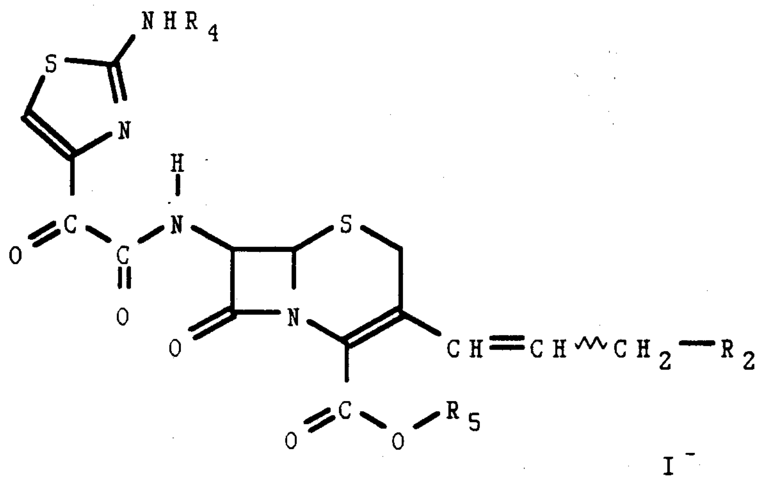

(XI)

pour conduire au produit de formule (X) telle que définie précédemment.

Les fonctions hydroxy protégées que peut porter le groupement $R_1$p sont choisies parmi les groupes acyloxy tel que par exemple formyloxy, acétoxy, propionyloxy, chloroacétoxy, bromoacétoxy, dichloroacétoxy, trichloroacétoxy, trifluoroacétoxy, méthoxyacétoxy, phénoxyacétoxy, benzoyloxy, benzoylformoxy, p-nitro benzoyloxy. On peut citer également les groupements éthoxycarbonyloxy, méthoxycarbonyloxy propoxycarbonyloxy, 2,2,2-trichloro éthoxycarbonyloxy, benzyloxycarbonyloxy, tert-butoxycarbonyloxy, 1-cyclopropyl éthoxycarbonyloxy, phtaloyloxy, butyryloxy, isobutyryloxy, valéryloxy, isovaléryloxy, oxalyloxy, succinyloxy et pivaloyloxy, phénylacétoxy, phénylpropionyloxy, mésyloxy, chlorobenzoyloxy, para-nitrobenzoyloxy, para-tert-butyl benzoyloxy, caprylyloxy, acryloyloxy, méthylcarbamoyloxy, phénylcarbamoyloxy, naphtylcarbamoyloxy.

On peut également citer les radicaux phénoxy, 4-chloro phénoxy, tolyloxy ou tert-butyl phénoxy, tétrahydropyrannyloxy, tétrahydrothiopyrannyloxy, méthoxytétrahydropyrannyloxy, trityloxy, benzyloxy, 4-méthoxy benzyloxy, benzhydryloxy, trichloroéthoxy, 1-méthyl 1-méthoxyéthoxy, ou les radicaux alkoxy alkoxy-méthyle tel que méthoxy éthoxy méthyle.

Les deux radicaux hydroxy peuvent encore être protégés en formant un radical méthylènedioxy, isopropylènedioxy, 1,1-cyclohexyl bis(oxy), diphénylméthylènedioxy, carbonate ou hydroxy borannylbis-(oxy).

Les fonctions hydroxy protégées que peut porter le groupement $R_1$p sont choisies de préférence parmi les groupes méthoxyéthoxyméthoxy, propionyloxyméthoxy, acétoxyméthoxy, butoxy, isobutoxy, tert-butoxy, pentoxy, hexyloxy, butyryloxyméthoxy, valéryloxyméthoxy, pivaloyloxyméthoxy, 2-acétoxy éthoxy, 2-propionyloxy éthoxy, 2-butyryloxy éthoxy, 2-iodoéthoxy, 2,2,2-trichloro éthoxy, vinyloxy, allyloxy, éthynyloxy, propynyloxy, benzyloxy, 4-méthoxy benzyloxy, 4-nitro benzyloxy, phényléthoxy, trityloxy, diphénylméthyloxy ou 3,4-diméthoxyphénoxy.

On préfère particulièrement le groupement 2-méthoxy éthoxyméthoxy (MEM-O).

Les restes de groupements esters facilement clivables que représentent $R_3$ et $R_5$, sont choisis parmi les groupements butyle, isobutyle, tert-butyle, pentyle, hexyle, méthoxyméthyle, éthoxyméthyle, isopropyloxyméthyle, alpha-méthoxy éthyle, alpha-éthoxy éthyle, méthylthiométhyle, éthylthiométhyle, isopropylthiométhyle, pivaloyloxyméthyle, acétoxyméthyle, propionyloxyméthyle, butyryloxyméthyle, isobutyryloxyméthyle, valéryloxyméthyle, isovaléryloxyméthyle, tert-butylcarbonyloxyméthyle, hexadécanoyloxyméthyle, pivaloyloxyméthyl, propionyloxyéthyle, isovaléryloxyéthyle, 1-acétoxy éthyle, 2-acétoxy éthyle, 1-propionyloxy éthyle, 2-propionyloxy éthyle, 1-butyryloxy éthyle, 2-butyryloxy éthyle, 1-(tert-butylcarbonyloxy) éthyle, 1-acétoxy propyle, 1-hexadécanoyloxy éthyle, 1-propionyloxy propyle, 1-méthoxycarbonyloxy éthyle, méthoxycarbonyloxyméthyle, 1-acétoxy butyle, 1-acétoxy hexyle, 1-acétoxy heptyle, phtalidyle, 5,6-diméthoxy phtalidyle, tert-butylcarbonylméthyle, vinyle, allyle, 2-chloro allyle, éthynyle, propynyle, méthoxycarbonylméthyle, benzyle, 4-méthoxy benzyle, 4-nitro benzyle, phénéthyle, trityle, diphénylméthyle, phényle, 4-chloro phényle, tolyle, tert-butyl phényle, 3,4-diméthoxy phényle, méthoxyéthoxyméthyle, diméthylaminoéthyle, cyanométhyle, tert-butoxycarbonylméthyle, 2,2-éthylènedioxy éthyle, cyanoéthyle, 2,2-diméthoxy éthyle ; 2-chloro éthoxyméthyle, (2-hydroxy éthoxy) éthyle, 2,3-époxy propyle, 3-diméthylamino 2-hydroxy propyle, 2-hydroxy éthyle, 2-méthylaminoéthoxyméthyle, (2-amino éthoxy) méthyle, 3-méthoxy 2,4-thiadiazol-5-yle, tétrahydropyrann-2-yle, 1-méthoxy 1-méthyl éthyle, 2-hydroxy 1-méthyl éthyle, isopropyle, carbamoylméthyle, chlorométhyle, 2-chloro éthyle, 2,2,2-trichloro éthyle, 2-iodo éthyle, acétyle, méthyle, 2-méthylthio éthyle, thiocyanatométhyle, 2-chloro 1-acétoxy éthyle, 2-bromo 1-acétoxy éthyle, 2-fluoro 1-acétoxy éthyle, 2-méthoxy 1-acétoxy éthyle, 2-méthyl 1-acétoxy propyle, 1-méthyl 1-acétoxy éthyle, 1-(méthoxyacétoxy) éthyle, 1-acétyl carbonyloxyéthyle, 1-hydroxy acétoxyéthyle, 1-(2-thiényl) carbonyloxyéthyle, 1-(2-furyl) carbonyloxyéthyle, 1-(5-nitro 2-furyl) carbonyloxyéthyle, 1-(2-pyrrolyl) carbonyloxyéthyle, 1-(propionyloxycarbonyloxy) éthyle, 1-(propoxycarbonyloxy) éthyle, 1-(isopropoxycarbonyloxy) éthyle, 1-(méthoxyéthoxycarbonyloxy) éthyle, 1-(allyloxycarbonyloxy) éthyle, isopropoxycarbonyl méthyle, 1-[(2,3-époxy propyl) oxycarbonyloxy] éthyle, 1-[(2-furyl) méthoxycarbonyloxy] éthyle, 1-[(2-fluoro éthoxy) carbonyloxy] éthyle, 1-(méthoxycarbonyloxy) propyle, 1-(méthoxycarbonyloxy) 1-méthyl éthyle, (méthoxycarbonyloxy) chlorométhyle, 1-(méthoxycarbonyloxy) 2-chloro éthyle, 1-(méthoxy carbonyloxy) 2-méthoxy éthyle, 1-(méthoxycarbonyloxy) allyle ou un reste 5-méthyl 2-oxo 1,3-dioxol-4-yle.

On préfère le radical diphénylméthyle pour $R_3$ et 4-méthoxy benzyle ou diphénylméthyle pour $R_5$.

Le groupement protecteur du radical amino que peut représenter $R_4$ ou que peut porter le groupement $R_1p$ peut être par exemple un groupe carbamoyle, méthyl carbamoyle, phénylcarbamoyle, naphtylcarbamoyle, ainsi que les thiocarbamoyles correspondants, un radical alkyle de 1 à 6 atomes de carbone substitué ou non substitué tel que, préférentiellement, trichloroéthyle, tert-butyle ou tert-amyle, un radical aralkyle tel que benzyle, 4-méthoxy benzyle, phénéthyle, trityle, 3,4-diméthoxy benzyle ou benzhydryle, un radical acyle aliphatique, aromatique ou hétérocyclique substitué ou non, tel que par exemple formyle, acétyle, propionyle, butyryle, isobutyryle, valéryle, isovaléryle, chloroacétyle, dichloroacétyle, trichloroacétyle, bromoacétyle, trifluoroacétyle benzoyle, toluolyle, naphtoyle, chlorobenzoyle, para-nitro benzoyle, para-tert-butyl benzoyle, phénoxyacétyle, caprylyle, décanoyle, acryloyle, phtaloyle, mésyle, phénylacétyle, phénylpropionyle, oxalyle, succinyle, pivaloyle, un radical alcoxycarbonyle ou cycloalcoxycarbonyle inférieur tel que par exemple, méthoxycarbonyle, éthoxycarbonyle, propoxycarbonyle, 1-cyclopropyléthoxycarbonyle, isopropoxycarbonyle, butoxycarbonyle, tert-butoxycarbonyle, pentoxycarbonyle, hexyloxycarbonyle, trichloroéthoxy carbonyle, un groupe aralcoxycarbonyle, tel que benzyloxycarbonyle.

On préfère le groupement trityle pour $R_4$ et benzyloxycarbonyle pour $R_1p$.

La liste ci-dessus n'est pas limitative, il est évident que d'autres groupements protecteurs des amines, groupements connus en particulier dans la chimie des peptides, peuvent également être utilisés.

Le groupement X peut être un radical hydroxy, un radical alkylsulfonyloxy tel que méthylsulfonyloxy, un radical arylsulfonyloxy tel que phényl ou tolylsulfonyloxy, ou un atome d'halogène tel que chlore, brome ou iode. Les valeurs hydroxy, chloro et bromo sont plus particulièrement préférées.

L'hydrolyse du dérivé de formule (III) est effectuée par des méthodes connues de l'homme du métier.

Le dérivé fonctionnel de l'acide de formule (VI) peut être par exemple un halogénure, un anhydride symétrique ou mixte, l'amide, l'azide ou un ester activé.

Comme exemple d'anhydride mixte on peut citer par exemple celui formé avec le chloroformiate d'isobutyle et celui formé avec le chlorure de pivaloyle et les anhydrides mixtes carboxylique-sulfonique formé par exemple avec le chlorure de para-toluène sulfonyle.

Comme exemple d'ester activé, on peut mentionner l'ester formé avec le 2,4-dinitrophénol et celui formé avec l'hydroxybenzothiazole.

Comme exemple d'halogénure, on peut citer le chlorure ou le bromure.

L'anhydride peut être formé in situ par action de carbodiimide N,N'-disubstitué, par exemple le N,N-dicyclohexylcarbodiimide.

La réaction d'acylation est conduite de préférence dans un solvant organique tel que le chlorure de méthylène. On peut cependant utiliser d'autres solvants tels que le tétrahydrofuranne, le chloroforme ou le diméthylformamide.

Lorsqu'on utilise un halogénure d'acide et de manière générale lorsqu'une molécule d'acide halohydrique est libérée au cours de la réaction, on réalise la réaction de préférence en présence d'une base telle que la soude, la potasse, les carbonates et carbonates acides de sodium ou de potassium, l'acétate de sodium, la triéthylamine, la pyridine, la morpholine ou la N-méthylmorpholine.

La température de réaction est en général inférieure ou égale à la température ambiante.

On peut également faire agir directement un produit de formule (VI) avec un produit de formule (VII) en présence d'un carbodiimide telle que le diisopropylcarbodiimide ou le 1-(3-diméthylamino propyl) 3-éthyl carbodiimide (EDC). Un exemple d'une telle préparation est donnée plus loin dans la partie expérimentale.

L'action des réactifs capable d'introduire le radical $R_2$ et conduire au produit de formule (X) est effectuée dans les conditions suivantes :

Lorsque Hal représente par exemple un atome de chlore, on peut effectuer in situ ou séparément une substitution de l'atome de chlore par un atome d'iode en présence d'iodure de sodium puis ajoute ensuite le réactif désiré, en présence ou non d'un solvant organique tel que le dichlorométhane, l'acétonitrile ou le tétrahydrofuranne.

On peut également faire agir directement sur le produit de formule (VIII) ou (IX), le réactif de formule $R_2$ désiré en présence de tétrafluoroborate d'argent.

L'isomérie des produits de formule (X) peut être différente de celle des produits de formule (VIII) ou (IX) utilisés au départ. Dans le cas où l'on isole l'isomère Z, on peut transformer cet isomère en isomère E selon les méthodes usuelles, notamment par action de l'iode.

Selon les valeurs de $R_3$, $R_4$, $R_5$ et $R_1p$, l'action sur le produit de formule (X) d'un ou plusieurs agents d'hydrolyse, d'hydrogénolyse ou de la thiourée a pour but d'éliminer le radical $R_4$ lorsque celui-ci représente un radical protecteur du radical amino, de transformer le groupement $R_1p$, en groupement $R_1$, lorsque celui-ci porte un groupement protecteur des radicaux hydroxyle et/ou d'éliminer les radicaux $R_3$ et $R_5$ lorsque ceux-ci représentent, parmi les groupements esters facilement clivables l'un de ceux que l'on désire éliminer.

Cependant, il est bien entendu possible d'éliminer $R_4$ et de transformer le groupement $R_1p$, en groupement $R_1$, lorsque celui-ci porte un groupement protecteur des radicaux hydroxyle sans toucher aux substituants $R_3$ et $R_5$ lorsque ceux-ci doivent être conservés. La nature des réactifs à mettre en jeu dans un tel cas est bien connu de l'homme du métier. On trouvera par exemple une description des différentes méthodes d'élimination des différents groupements protecteurs dans le brevet français B.F. 2.499.995. Un exemple de telles réactions est donné plus loin dans la partie expérimentale.

Etant donné la nature des groupements protecteurs préférés que l'on utilise : trityle pour $R_4$, 2-méthoxy éthoxy méthyle pour protéger les fonctions hydroxy, et benzyloxycarbonyle pour protéger la fonction amino de $R_1$ diphénylméthyle pour $R_3$ et 4-méthoxy benzyle ou diphénylméthyle pour $R_5$, on utilise de préférence l'acide trifluoroacétique sans solvant ou dans un solvant tel que l'anisole ou un mélange de solvants tels que anisole/chlorure de méthylène. On obtient alors un sel avec l'acide trifluoroacétique. On peut revenir à la base libre par action d'une base telle que le carbonate de triéthylamine.

La salification des produits peut être effectuée selon les méthodes usuelles ; elle peut par exemple, être obtenue par action, sur un produit sous forme acide ou sur un solvat, par exemple le solvat éthanolique, ou un hydrate de cet acide, d'une base minérale telle que l'hydroxyde de sodium ou de potassium, le carbonate ou le carbonate acide de sodium ou de potassium. On peut également utiliser les sels d'acides minéraux tels que le phosphate trisodique. On peut également faire appel à des sels d'acides organiques tels que par exemple, les sels de sodium d'acides carboxyliques aliphatiques, linéaires ou ramifiés, saturés ou insaturés de 1 à 18 et de préférence de 2 à 10 atomes de carbone. Les chaînes aliphatiques de ces acides peuvent être interrompues par un ou plusieurs hétéroatomes tels que l'oxygène ou le soufre ou substituées par des radicaux aryle tels que phényle, thiényle ou furyle, par un ou plusieurs radicaux hydroxyles ou par un ou plusieurs atomes d'halogène tels que fluor, chlore ou brome, préférentiellement chlore, par un ou plusieurs radicaux carboxyliques ou alkoxycarbonyles inférieurs, de préférence méthoxycarbonyle, éthoxycarbonyle ou propyloxycarbonyle, par un ou plusieurs radicaux aryloxy, de préférence phénoxy.

De plus, on peut utiliser comme acides organiques, des acides aromatiques suffisamment solubles comme, par exemple, des acides benzoïques substitués, de préférence par des radicaux alkyles inférieurs.

Comme exemples de tels acides organiques, on peut mentionner les acides formique, acétique, acrylique, butyrique, adipique, isobutyrique, n-caproïque, isocaproïque, chloropropionique, crotonique, phényl acétique, (2-thiényl) acétique, (3-thiényl) acétique, (4-éthyl phényl) acétique, glutarique, l'ester monoéthylique de l'acide adipique, les acides hexanoïque, heptanoïque, décanoïque, oléïque, stéarique, palmitique, 3-hydroxy propionique, 3-méthoxy propionique, 3-méthyl thiobutyrique, 4-chloro butyrique, 4-phényl butyrique, 3-phénoxy butyrique, 4-éthyl benzoïque, 1-propyl benzoïque.

On utilise cependant de préférence comme sels de sodium, l'acétate de sodium, le 2-éthyl hexanoate de sodium ou le diéthyl acétate de sodium.

La salification peut également être obtenue par action d'une base organique comme la triéthylamine, la diéthylamine, la triméthylamine, la propylamine, la N,N-diméthyl éthanolamine, le tris[(hydroxyméthyl) amino] méthane, la méthylamine, l'éthanolamine, la pyridine, la picoline, la dicyclohexyl amine, la morpholine et la benzylamine.

Elle peut également être obtenue par action de l'arginine, de la lysine, de la procaïne, de l'histidine, de la N-méthyl glucamine.

Cette salification est réalisée de préférence dans un solvant ou un mélange de solvants tels que l'eau, l'éther éthylique, le méthanol, l'éthanol ou l'acétone.

Les sels sont obtenus sous forme amorphe ou cristallisée selon les conditions réactionnelles employées.

Les sels cristallisés sont préparés de préférence en faisant réagir les acides libres avec l'un des sels des acides carboxyliques aliphatiques mentionnés ci-dessus, de préférence, avec l'acétate de sodium.

La salification des produits par les acides minéraux ou organiques est effectuée dans les conditions usuelles.

L'estérification éventuelle des produits est effectuée dans les conditions classiques. On opère en général en faisant réagir l'acide de formule (I) ou un dérivé fonctionnel avec un dérivé de formule :

Z-Re

dans laquelle Z représente un radical hydroxyle ou un atome d'halogène tel que le chlore, le brome, l'iode et Re désigne le groupement ester à introduire, groupement dont une liste non exhaustive figure ci-dessus. Dans certains cas, il peut être avantageux d'effectuer une estérification sur un produit dont l'amine et/ou les groupements réactionnels présents sur l'oxyimino sont bloqués avant d'enlever le groupement protecteur de l'amine et du groupement réactionnel présents sur l'oxyimino.

Les produits de formule (I) comportent plusieurs carbones asymétriques. Dans le noyau cephème, qui comporte deux carbones asymétriques, les deux carbones sont dans la configuration R. Par ailleurs, le radical présent sur la fonction oxyimino comporte également un carbone asymétrique qui peut être sous forme R ou S ou sous forme d'un mélange R + S. La séparation des deux diastéréoisomères peut être effectuée par les moyens connus de l'homme du métier, par exemple par chromatographie.

Les dérivés de formule (II) sont nouveaux. A ce titre, et notamment à titre de produits intermédiaires nécessaires à la préparation des produits de formule (I), ils constituent l'un des objets de l'invention. Ces dérivés peuvent être obtenus au départ d'un dérivé de formule (XII) :

$R_1$-CHO (XII)

dont, si nécessaire, l'on protège les fonctions réactives, pour obtenir un dérivé de formule (XIII) :

$R_1$p-CHO (XIII)

dérivé de formule (XII) ou (XIII) que l'on transforme en dérivé alpha-hydroxy acide de formule (XIV) :

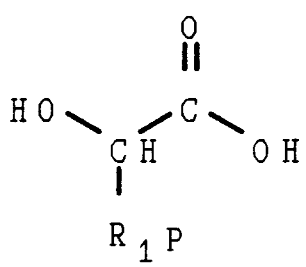

**(XIV)**

lequel peut être, si désiré, transformé en dérivé halogéné ou alkyl ou arylsulfonylé correspondant, puis que l'on estérifie.

Les dérivés de formule (II) dans laquelle X représente un atoms d'halogène peuvent de manière préférentielle être obtenus par un procédé selon lequel l'on traite un aldéhyde de formule (XII) ou (XIII) par le malononitrile, pour obtenir le dérivé de formule (XV) :

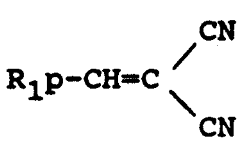

**(XV)**

que l'on soumet à l'action d'un réactif d'oxydation pour obtenir l'époxyde de formule (XVI) :

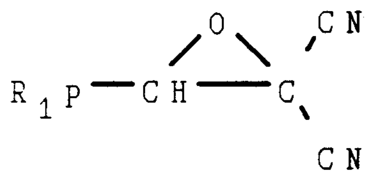

(XVI)

que l'on traite par un acide halohydrique pour obtenir l'acide de formule (XVII) :

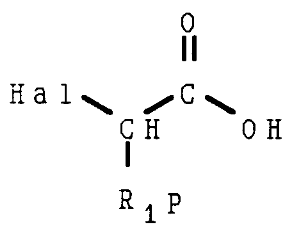

(XVII)

que l'on estérifie.

Les dérivés de formules (III), (IV), (VI), (VIII), (IX) et (X), ainsi que les dérivés de formules (XIV), (XV), (XVI) et (XVII) sont également nouveaux. A ce titre et notamment à titre de produits intermédiaires nécessaires à la préparation des produits de formule (I), ils constituent l'un des objets de l'invention.

Les dérivés de formule (II) dans laquelle X représente un radical hydroxy peuvent encore être préparés par un procédé selon lequel on soumet un composé de formule (XVIII) :

$R_1p\text{-}CH_2\text{-}COOR_3$ (XVIII)

à l'action d'un agent d'oxydation, pour obtenir un dérivé cétonique de formule (XIX) :

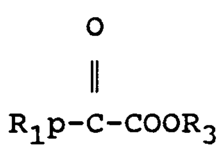

(XIX)

que l'on réduit en l'alcool secondaire correspondant de formule (II). Un exemple figure ci-après dans la partie expérimentale.

Les composés de formule (XII) sont connus, par exemple, par les références CA 111P194772, 108P150482, 101P38342, 97P215983, 95P203734 ou encore J. Am. Chem. Soc. 73 - 2956 (1951), ou préparables par des méthodes connues de l'homme du métier, à partir des composés décrits dans ces références.

Les composés de formule (XVIII) et l'acide correspondant sont quant à eux décrits dans la demande de brevet européen 136721, ainsi que dans J. of Antibiotics vol. 36 n° 8 p. 1020 (1983). Les composés de formule (XVIII) sont de manière générale, préparables à partir de l'acide, par des méthodes connues de l'homme du métier.

Les produits de formule générale (I) possèdent une très bonne activité antibiotique sur les bactéries gram (+) telles que les staphylocoques, les streptocoques et notamment sur les staphylocoques pénicillino-résistants. Leur efficacité sur les entérobactéries productrices de $\beta$-lactamases chromosomales ou plasmidiques, est particulièrement remarquable.

Ces propriétés rendent aptes lesdits produits ainsi que leurs sels d'acides pharmaceutiquement acceptables à être utilisés comme médicaments dans le traitement des infections à germes sensibles et notamment dans celui des staphylococcies, telles que septicémies à staphylocoques, staphylococcies malignes de la face ou cutanée, pyodermites, plaies septiques ou suppurantes, anthrax, phlegmons, érésipèles, staphylococcies aiguës primitives ou post grippales, bronchopneumonies, suppurations pulmonaires.

Ces produits peuvent également être utilisés comme médicaments dans le traitement des colibacilloses et infections associées, dans les infections à proteus, à klebsiella et à salmonella et dans d'autres infections provoquées par des bactéries à gram (-).

La présente invention a donc également pour objet, à titre de médicaments et notamment de médicaments antibiotiques, les produits de formule (I) tels que définis ci-dessus ainsi que leurs sels d'acides pharmaceutiquement acceptables.

L'invention a plus particulièrement pour objet à titre de médicaments les produits de formule (I) telle que décrite ci-dessus dans laquelle $R_2$ est choisi parmi les radicaux quinoléinium, isoquinoléinium, 4-(méthylthio) pyridinium thièno[2,3-b]pyridinium, imidazo (1,2-a) pyridinium et 6,7-dihydro 5H-pyrindinium et ceux dans laquelle $R_1$ représente un radical de formule (K) dans laquelle $R'_1$ représente un radical cyano, carboxy ou alkoxycarbonyle.

L'invention a spécialement pour objet, à titre de médicaments et notamment de médicaments antibiotiques, le produit dont le nom suit :

- le sel interne de [6R-[3-(E) 6alpha, 7béta-(Z)]] 1-[3-[7-[[(2-amino 4-thiazolyl) [[carboxy (5-cyano 3,4-dihydroxy 2-thiényl) méthoxy] imino] acétyl] amino] 2-carboxy 8-oxo 5-thia 1-azabicyclo[4,2,0]oct-2-èn-3-yl] 2-propényl] quinoléinium ainsi que leurs sels pharmaceutiquement acceptables.

L'invention s'étend aux compositions pharmaceutiques renfermant, comme principe actif, au moins un des médicaments définis ci-dessus.

Ces compositions peuvent être administrées par voie buccale, rectale, parentérale, notamment intramusculaire ou par voie locale en application topique sur la peau et les muqueuses.

Les produits de formule (I) et notamment ceux dans laquelle A représente un ester clivable peuvent être administrés par voie orale.

Les compositions selon l'invention peuvent être solides ou liquides et se présenter sous les formes pharmaceutiques couramment utilisées en médecine humaine comme par exemple, les comprimés simples ou dragéifiés, les gélules, les granulés, les suppositoires, les préparations injectables, les pommades, les crèmes, les gels ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent notamment se présenter sous forme d'une poudre destinée à être dissoute extemporanément dans un véhicule approprié, par exemple, de l'eau stérile apyrogène.

La dose administrée est variable selon l'infection traitée, le sujet en cause, la voie d'administration et le produit considéré. Elle peut être, par exemple, comprise entre 0,250 g et 4 g par jour, par voie orale chez l'homme, avec le produit décrit à l'exemple 1 ou encore comprise entre 0,500 g et 1 g trois fois par jour par voie intramusculaire.

Les produits de formule (I) peuvent également être utilisés comme désinfectants des instruments chirurgicaux.

Les produits de formules (V) et (VII) sont connus dans la littérature, notamment dans les demandes de brevets belge BE864828 et européenne EP0333154.

Les exemples suivants illustrent l'invention sous toutefois la limiter.

**PREPARATION 1 : [5-cyano 3,4-bis[(2-méthoxy éthoxy) méthoxy] thiényl chloro acétate de diphénylméthyle**

STADE A : 3,4-bis[(2-méthoxy éthoxy) méthoxy] 5-cyano 2-éthoxycarbonyl thiophène

On mélange sous gaz inerte à 0°C, 92 g de 3,4-dihydroxy 5-cyano 2-éthoxycarbonyl thiophène (décrit dans la demande japonaise n° J57116064), 860 $cm^3$ de chlorure de méthylène, ajoute lentement 153 $cm^3$ de diisopropyléthylamine, puis 102 $cm^3$ de chlorure de méthoxyéthoxyméthyle. Après 45 mn, on lave par une solution 0,1 N d'acide chlorhydrique puis par une solution 1M de bicarbonate de sodium et enfin à l'eau saturée de chlorure de sodium. On sèche et évapore le solvant. On obtient 161 g de produit attendu utilisé tel quel dans l'étape suivante :

Spectre RMN ($CDCl_3$, 300 MHz, ppm) :

1,36 (t), 4,34 (q) $CO_2Et$ ;
3,36 (s), 3,38 (s) 2-$OCH_3$ ;
3,50 à 3,63 (m) (4H), 3,94 (m) (4H) $O-(CH_2)_2-O$ ;
5,36 (s), 5,42 (s)

$$2 \quad =\overset{|}{C}-O-\underline{CH_2}-O \;;$$

Spectre IR ($CHCl_3$)

Absorptions à 2225 $cm^{-1}$ (C≡N conjugué) ; 1720 $cm^{-1}$ (C = O) ; 1554, 1490 $cm^{-1}$ (système conjugué).

Stade B : 3,4-bis[(2-méthoxy éthoxy) méthoxy] 5-cyano 2-hydroxyméthyl thiophène

On agite sous gaz inerte un mélange de 161 g de produit obtenu au stade A et 1 l de tétrahydrofuranne, et ajoute à 5 °C en 5 h., 262 g de triterbutoxy alumino hydrure de lithium. On laisse remonter la température et maintient sous agitation pendant 36 h.. On concentre la suspension et la verse dans un mélange de 1,2 l de solution aqueuse saturée de chlorure d'ammonium, 1,5 l d'eau et 0,5 l d'acétate d'éthyle. On agite pendant 18 h., filtre, lave le filtrat par une solution aqueuse saturée de chlorure de sodium, le sèche et évapore le solvant. On obtient 108 g de produit attendu utilisé tel quel pour le stade suivant.

Stade C : 3,4-bis[(2-méthoxy éthoxy) méthoxy] 5-cyano 2-formyl thiophène

On ajoute aux 108 g d'alcool obtenu au stade B, 450 $cm^3$ de chlorure de méthylène. On refroidit à 5 °C puis ajoute lentement 497 g de dioxyde de manganèse. Après disparition totale du produit de départ, on filtre et rince le filtre à l'acétate d'éthyle. On concentre le filtrat à sec et chromatographie le résidu sur silice en éluant aux mélanges chlorure de méthylène-acétate d'éthyle 98/2, 97/3 puis 96/4. On obtient 67 g de produit attendu.

Spectre RMN ($CDCl_3$, 300 MHz, ppm) :

3,35 (s) (3H), 3,38 (s) (3H) les $O-CH_3$ ;
3,58 (m) (4H), 3,93 (m) (4H) les $O-(CH_2)_2-O$ ;
5,37 (s) (2H), 5,44 (s) (2H) les $O-CH_2-O$ ;
10,04 (s) (1H) CHO

Spectre IR ($CHCl_3$)

Absorptions à 2220 $cm^{-1}$ (C≡N) ; 1670 $cm^{-1}$ (C = O, aldéhyde conjugué) ; 1582, 1550, 1485 $cm^{-1}$ (hétérocycle).

Stade D : [5-cyano 3,4-bis[(2-méthoxy éthoxy) méthoxy] thiényl chloro acétate de diphénylméthyle

On mélange 63,6 g de produit obtenu au stade C, 1 l de toluène, 15,8 g de malodinitrile et du tamis moléculaire 4Å. On refroidit à 5 °C, et ajoute 1,8 $cm^3$ de pipéridine, puis agite pendant 40 mn.

On ajoute au mélange réactionnel, en 5 mn et sans dépasser 11 °C, 74 $cm^3$ de terbutylhydroperoxyde en solution 3M dans le toluène. Après 20 mn, on filtre le mélange, rince le filtre à l'acétate d'éthyle et évapore le solvant. On recueille 82 g d'huile constituée par un dicyanoépoxyde.

On reprend le produit dans 1,8 l de tétrahydrofuranne, puis ajoute à la solution, en 7 mn environ, 240 $cm^3$ d'acide chlorhydrique 1N. On agite à température ambiante pendant 1h.

On ajoute au mélange réactionnel, en 12 mn environ, 870 $cm^3$ d'une solution à 0,38 mole/l de diphényldiazométhane dans l'éther éthylique. On agite pendant 2 h à température ambiante, puis extrait à

l'éther éthylique. On lave à la soude 1N puis à l'eau saturée en chlorure de sodium. On sèche la phase organique et évapore le solvant, puis chromatographie le résidu sur silice en éluant avec des mélanges cyclohexane-acétate d'éthyle (4/1), (3/1) puis (2/1). On obtient 68 g de produit attendu.

Spectre RMN ($CDCl_3$, 250 MHz, ppm) :

3,31 (s) (3H), 3,36 (s) (3H) les $O-CH_3$ ;
3,48 (m), 3,58 (m), 3,82 (m),
3,92 (m) (8H) les $O-(CH_2)_2-O$ ;
5,17 (syst. AB), 5,41 (s) (2H) les $O-CH_2-O$ ;
5,98 (s) (1H)

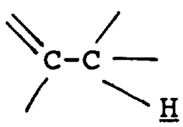

;

6,91 (s) (1H) $CO_2$-CH-$\Phi_2$ ;
7,34 (m) (10H) les phényles.

Spectre IR ($CHCl_3$)

Absorptions à 2221 $cm^{-1}$ (C≡N) ; 1746 $cm^{-1}$ (C=O), 1603, 1589, 1496 $cm^{-1}$ (aromatique-hétéroatome).

**EXEMPLE 1 : Sel interne de [6R-[3-(E) 6alpha, 7béta-(Z)]] 1-[3-[7-[[(2-amino 4-thiazolyl) [[carboxy (5-cyano 3,4-dihydroxy 2-thiényl) méthoxy] imino] acétyl] amino] 2-carboxy 8-oxo 5-thia 1-azabicyclo-[4,2,0]oct-2-èn-3-yl] 2-propényl] quinoléinium**

Stade A : [5-cyano 3,4bis[(2-méthoxy éthoxy) méthoxy] thiényl] phtalimidoxy acétate de diphénylméthyle

On mélange 13,1 g de N-hydroxy phtalimide, 6,55 g de bicarbonate de sodium et 400 $cm^3$ d'eau, agite pendant 2 h. puis ajoute 25 g de [5-cyano 3,4-bis[(2-méthoxy éthoxy) méthoxy] thiényl chloroacétate de diphénylméthyle dans 400 $cm^3$ de dichloréthane, puis 0,89 g de chlorure de triéthyl benzylammonium. On agite pendant 13 h. à température ambiante, puis extrait au dichloréthane. On lave la phase organique à l'eau additionnée de chlorure de sodium, sèche et évapore le solvant. On purifie le résidu par chromatographie sur silice en éluant au mélange cyclohexane-acétate d'éthyle (6-4) puis (1-1) et obtient 15,4 g de produit attendu.

Spectre RMN ($CDCl_3$, 300 MHz, ppm) :

3,28 (s) (3H), 3,36 (s) (3H) $-O-CH_3$ ;
3,46 (m) (2H), 3,58 (m) (2H),
3,78 à 3,95 (m) (4H) $-O-(CH_2)_2-O$ ;
5,21 (AB) (2H), 5,37 (AB) (2H) $O-CH_2-O$ ;
6,26 (s) (1H)

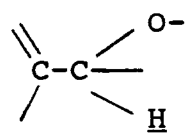

;

6,96 (s) (1H) $-CO_2$-CH-$\Phi_2$ ;
7,2 à 7,4 (m) (10H), 7,75 (m) (4H) H phtalimide.

Spectre IR ($CHCl_3$)

Absorptions à 2222 $cm^{-1}$ : -C≡N ; 1796 et 1740 $cm^{-1}$ C=O, 1610-1580 $cm^{-1}$ et 1496 $cm^{-1}$ : système conjugué + aromatique.

Stade B : Aminoxy [5-cyano 3,4-bis[(2-méthoxy éthoxy) méthoxy] thiényl] acétate de diphénylméthyle.

On dissout sous azote 5,06 g du produit préparé au stade précédent dans 72 $cm^3$ de méthanol, refroidit à -11°C puis ajoute 0,35 $cm^3$ d'hydrate d'hydrazine, maintient à la même température pendant 15 mn, rajoute 0,02 $cm^3$ d'hydrate d'hydrazine, puis après 30 mn, de nouveau 0,04 $cm^3$. On maintient sous agitation pendant 45 mn. On obtient ainsi le produit attendu en solution, laquelle est utilisée telle quelle pour le stade suivant.

Stade C : Acide [[[2-[5-cyano 3,4-bis[(2-méthoxy éthoxy) méthoxy] thiényl] 2-oxo 2-(diphénylméthoxy) éthyl] oxy] imino] [2-[(triphénylméthyl) amino] thiazol-4-yl] acétique

On ajoute à la solution obtenue au stade précédent 3 g d'acide oxo [2-[(triphénylméthyl) amino] thiazol-4-yl] acétique (décrit dans la demande de brevet belge N° 864828) et laisse remonter la température.

On ajoute 10 $cm^3$ de chlorure de méthylène puis essore les cristaux formés. On concentre le filtrat à sec et chromatographie le résidu sur silice en éluant au mélange chlorure de méthylène-méthanol (95-5) et récupère de nouveau le produit. On obtient au total 5,5 g de produit attendu brut.

Stade D : 7béta-[[[[[2-[5-cyano 3,4-bis[(2-méthoxy éthoxy) méthoxy] thiényl] 2-oxo 2-(diphénylméthoxy) éthyl] oxy] imino] [2-[(triphénylméthyl) amino] thiazol-4-yl] acétyl] amino] 3-[(Z)-3-chloro 1-propényl] 8-oxo 5-thia 1-azabicyclo[4,2,0]oct-2-èn-2-carboxylate de 4-méthoxy benzyle

On mélange à 0°C 2,44 g de chlorhydrate de 7béta-amino 3-[(Z)-3-chloro 1-propényl] 8-oxo 5-thia 1-azabicyclo[4,2,0]oct-2-èn-2-carboxylate de 4-méthoxy benzyle (décrit dans la demande de brevet européen N° 0333 154), 5,5 g du produit préparé au stade précédent, 60 $cm^3$ de chlorure de méthylène puis on ajoute 1,75 g de 1-(3-diméthylamino propyl) 3-éthyl carbodiimide (EDC), et agite pendant 45 mn en laissant remonter la température. On traite avec 100 $cm^3$ d'hydrogénophosphate de potassium 0,5M puis lave avec une solution de chlorure de sodium. On sèche et évapore le solvant. On recueille 8,5 g de produit attendu. (Rf = 0,42 [éluant : chlorure de méthylène-acétate d'éthyle (9-1)] que l'on peut purifier par chromatographie sur silice en éluant au mélange cyclohexane-acétate d'éthyle (1-1).

Spectre RMN ($CDCl_3$, 300 MHz, ppm) :

3,20 à 3,34 -O-$CH_3$, S-$CH_2$- ;
3,43 (m), 3,59 (m), 3,92 (m),
3,45 à 3,90, 4,14 (m) (1H) -O-$CH_2$-$CH_2$-O, -CH-$CH_2$-X
5,02 (m) $H_6$ ;
5,86 (m) $H_7$ ;
5,40 à 5,50 Φ-CH-OCO, O-$CH_2$-O ;
5,78 (m) $CH_2$-CH=CH-(ΔZ) ;
6,20 à 6,35 $CH_2$-CH=CH-(ΔZ) ;
6,77 (s/d) $H_5$ thiazole ;
6,91 (m) (4H) Aromatiques, -$CO_2$-CH-Φ, NH-CO-

Spectre IR ($CHCl_3$)

Absorptions à 3404 $cm^{-1}$ : =C-NH- ; 2221 $cm^{-1}$ : -C≡N ; 1791, 1730 et 1684 $cm^{-1}$ : β-lactame ; 1613, 1587, 1526, 1517 et 1496 $cm^{-1}$ : C=C aromatique, hétéroatome et amide II.

Stade E : 7béta-[[[[[2-[5-cyano 3,4-bis[(2-méthoxy éthoxy) méthoxy] thiényl] 2-oxo 2-(diphénylméthoxy) éthyl] oxy] imino] [2-[(triphénylméthyl) amino] thiazol-4-yl] acétyl] amino] 3-[(Z)-3-iodo 1-propényl] 8-oxo 5-thia 1-azabicyclo[4,2,0]oct-2-èn-2-carboxylate de 4-méthoxy benzyle

On agite pendant 1 h 10 mn à température ambiante 1,5 g du produit obtenu après chromatographie au stade précédent, 20 $cm^3$ d'acétone et 0,5 g d'iodure de sodium en présence de 30 mg d'iode. Après élimination du solvant, on reprend le résidu dans le chlorure de méthylène, lave la phase organique par une solution de thiosulfate de sodium puis par une solution de chlorure de sodium, sèche, élimine le solvant et chromatographie le résidu sur silice en éluant au mélange cyclohexane-acétate d'éthyle (1-1). On recueille 0,9 g de produit iodé attendu.

Stade F : Iodure de 1-[3-[7béta-[[[[2-[5-cyano 3,4-bis[(2-méthoxy éthoxy) méthoxy] thiényl] 2-oxo 2-(diphénylméthoxy) éthyl] oxy] imino] [2-[(triphénylméthyl) amino] thiazol-4-yl] acétyl] amino] 2-[(4-méthoxy benzyloxy) carbonyl] 8-oxo 5-thia 1-azabicyclo[4,2,0][oct-2-en-3-yl] 2-propènyl] quinoléinium

On dissout 0,450 g du produit obtenu au stade précédent dans 5 $cm^3$ de chlorure de méthylène et ajoute 185 $\mu$l de quinoléine. On agite, évapore le solvant et maintient l'agitation pendant 1 h 20 mn. On reprend dans l'éther, cristallise, essore et chromatographie sur silice en éluant par le mélange chlorure de méthylène-méthanol (95-5). On recueille ainsi 0,260 g de produit attendu. Rf = 0,37 [éluant: chlorure de méthylène-méthanol (90-10)].

Stade G : Sel interne de [6R-[3(E), 6alpha, 7béta(Z)]] 1-[3-[7-[[(2-amino 4-thiazolyl) [[carboxy (5-cyano 3,4-dihydroxy 2-thiényl) méthoxy] imino] acétyl] amino] 2-carboxy 8-oxo 5-thia 1-azabicyclo[4,2,0]oct-2-en-3-yl] 2-propènyl] quinoléinium

On agite à température ambiante pendant 2 heures et 45 mn un mélange contenant 0,255 g du produit préparé au stade précédent et 3 $cm^3$ d'acide trifluoroacétique contenant 10 % d'anisole. Après ajout d'éther, isopropylique, filtration, lavage et sèchage pendant 16 heures sous pression réduite à température ambiante, on isole 0,118 g du sel interne recherché.

Analyse RMN du proton (DMSO 300 MHz en ppm)

Structure R/S ; Δ3 syn ; mélange ΔE/ΔZ ≃ 70/30.
5,18 (m) et 5,30 (m) (1H) : $H_6$ ;
5,65/5,90 (<4H) : $H_7$,

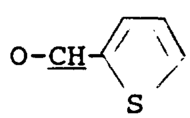 ,

=CH-$CH_2$-$N^+$ et =CH-$CH_2$ (ΔZ)
6,39 (m) (0,7H) : C=CH-$CH_2$ (ΔE) ;
6,61 (d,J=11) (0,3H) : C=CH-$CH_2$ (ΔZ) ;
6,70 (s) et 6,74 (s) (1H) : $H_5$ thiazole ;
6,95 (d, J=15,5) : =C-CH-CH (ΔE) ;
8,07 (m) (1H), 8,29 (m) (2H), 8,51 (m) (2H), 9,34 (m) (1H) et 9,57 (m) (1H) : H du quinoléinium ;
11,6 (l) : =C-NH-CH.

Spectre IR (nujol)

Absorption générale OH/NH, 2215 $cm^{-1}$ : C≡N conjugué, 1778 $cm^{-1}$, $\beta$-lactame, 1672 $cm^{-1}$ : autre C=O.

**PREPARATION 2 : $\alpha$-bromo [5-(terbutoxycarbonyl) amino] 3-(1,2,4-thiadiazole) acétate de diphénylméthyle.**

Stade A : [5-(terbutoxycarbonyl) amino] 3-(1,2,4-thiadiazole) acétate de diphénylméthyle.

On mélange à température ambiante 7,25 g d'acide [5-(terbutoxycarbonyl) amino] 3-(1,2,4-thiadiazole)] acétique dans 90 $cm^3$ de chlorure de méthylène et 49 $cm^3$ de méthanol. On refroidit la solution à 0°C et ajoute goutte à goutte 93,3 $cm^3$ de diphényldiazométhane en solution 0,3M dans l'éther. On laisse revenir à

température ambiante en 2 heures et demie, évapore les solvants sous pression réduite, obtient 13,66 g de produit brut que l'on chromatographie sur silice (éluant : chlorure de méthylène-acétonitrile 98-2). On récupère 8,38 g de produit attendu.

Spectre RMN ($CDCl_3$ 300 MHz en ppm)

1,52 (s) terbutyle
4,06 (s) $CH_2$-$CO_2$
6,92 (s) $CO_2$-$\underline{CH}$-$\Phi_2$
7,26 (s) phényles
10,20 (sl) NH

Stade B : α-bromo [5-(terbutoxycarbonyl) amino] 3-(1,2,4-thiadiazole) acétate de diphénylméthyle.

On ajoute 146 mg de brome (47 µl) à 390 mg d'ester préparé au stade A en solution dans 6 $cm^3$ d'acide acétique. On agite pendant 2 heures et demie à température ambiante, évapore le solvant, reprend le résidu dans le chlorure de méthylène, le lave avec une solution aqueuse de bicarbonate de sodium à 10%, sèche et évapore les solvants. On récupère 390 mg de produit brut que l'on chromatographie sur silice (éluant : chlorure de méthylène). On obtient 190 mg de produit attendu.

Spectre RMN ($CDCl_3$ 250 MHz en ppm)

1,55 terbutyle
6,95 phényles
7,2 à 7,4 -C$\underline{H}$-Br
9,23 NH

**EXEMPLE 2 : Sel interne de [6R-[3-(E) 6alpha, 7béta-(Z)]] 1-[3-[7-[[(2-amino 4-thiazolyl) [[carboxy (5-amino 1,2,4-thiadiazol-3-yl) méthoxy] imino] acétyl] amino] 2-carboxy 8-oxo 5-thia 1-azabicyclo-[4,2,0]oct-2-èn-3-yl] 2-propényl] quinoléinium**

Stade A : α-phtalimidoxy [5-(terbutoxycarbonyl) amino] 3-(1,2,4-thiadiazole) acétate de diphénylméthyle.

On agite pendant 1 heure sous atmosphère inerte 130 mg de bicarbonate de sodium, 252 mg d'hydroxyphtalimide dans 3 $cm^3$ d'eau puis ajoute goutte à goutte 390 mg de bromo ester préparé comme indiqué dans la préparation 2 puis 17,6 mg de chlorure de triéthylbenzylammonium. On agite vivement pendant 20 heures, ajoute 10 à 20 $cm^3$ de chlorure de méthylène, décante, extrait au chlorure de méthylène, sèche, évapore le solvant. On obtient 426 mg de produit brut que l'on purifie par chromatographie sur silice (éluant : chlorure de méthylène-acétate d'éthyle 98-2) et obtient 169 mg de produit attendu.

Spectre RMN ($CDCl_3$ 300 MHz en ppm)

1,56 (s) terbutyle
5,98 (s) -CH-COO
7,07 (s) $CO_2$-$\Phi_2$
7,15 à 7,35 phényles
7,72 (m)-7,76 (m) phtalimide
8,53 (sl) -NH-

Stade B : α-aminoxy [5-(terbutoxycarbonyl) amino] 3-(1,2,4-thiadiazole) acétate de diphénylméthyle.

On refroidit à 0 °C, sous atmosphère inerte 58 mg de phtalimido ester préparé au stade précédent dans 1 $cm^3$ de chlorure de méthylène puis ajoute 5,5µl d'hydrate d'hydrazine, agite 30 minutes, filtre, rince le précipité au chlorure de méthylène, évapore le solvant sous pression réduite à température ambiante et récupère 58 mg de produit brut que l'on chromatographie sur silice (éluant :cyclohexane-acétate d'éthyle 7-3). On obtient 26 mg de produit attendu.

Spectre RMN ($CDCl_3$ 300 MHz en ppm)

1,55 (sl) terbutyle
6,22 (s) -CH-COO
6,90 à 7,30 phényles
6,96 ou 7,10 O-CH-Φ
3,10 H mobiles

Stade C : Iodure de 1-[3-[7-[(triphényl) amino] 4-thiazolyl] (2-diphénylméthoxy) carbonyl] [5-(terbutoxycarbonyl) amino] (1,2,4-thiadiazol-3-yl) méthoxy] imino] acétyl] amino] 2-[(4-méthoxy) benzyloxycarbonyl] 8-oxo 5-thia 1-azabicyclo[4,2,0]oct-2-èn-3-yl] 2-propényl] quinoléinium

On mélange sous atmosphère inerte à température ambiante 65,4 mg d'iodure de 1-[3-[7β-[[oxo [2-(triphénylméthyl) amino] 4-thiazolyl] acétyl] amino] 2-[(4-méthoxy) benzyloxycarbonyl] 8-oxo 5-thia 1-azabicyclo [4,2,0] oct-2-èn-3-yl] 2-propényl] quinoléinium, 1 $cm^3$ de méthanol et 0,3 $cm^3$ de chlorure de méthylène. On ajoute 36 mg du dérivé aminoxy préparé comme au stade précédent dans 0,5 $cm^3$ de chlorure de méthylène, puis 12,5 mg d'acide paratoluène sulfonique. On agite 20 heures à température ambiante, évapore le solvant sous pression réduite à température ambiante puis reprend dans l'éther. On agite 15 minutes, filtre le précipité, le sèche sous pression réduite pendant 1 heure et recueille 83 mg de produit brut que l'on utilise tel quel pour la suite de la synthèse.

Stade D : Sel interne de [6R-[3-(E) 6alpha, 7béta-(Z)]] 1-[3-[7-[[(2-amino 4-thiazolyl) [[carboxy(5-amino 1,2,4-thiadiazol-3-yl) méthoxy] imino] acétyl] amino] 2-carboxy 8-oxo 5-thia 1-azabicyclo[4,2,0]oct-2-èn-3-yl] 2-propényl] quinoléinium.

On agite 3 heures à température ambiante 83 mg du produit brut ci-dessus dans 0,8 $cm^3$ d'une solution d'acide trifluoroacétique à 10% d'anisole. On filtre, rince à l'acide trifluoroacétique puis ajoute de l'éther éthylique (environ 2 $cm^3$) et agite pendant 30 minutes. On filtre, rince à l'éther et sèche le précipité sous pression réduite pendant 16 heures à température ambiante. On obtient 34 mg de produit brut attendu.

Spectre RMN ($CDCl_3$ 300 MHz en ppm)

5,26 (d) $H_6$
5,48 (d,d) après échange 6,53 (d) $H_7$
6,09 (dt) ΔE $CH=C\underline{H}-CH_2$
6,53 $C\underline{H}=CH-CH_2$
6,75 (s) $H_5$ du thiazole
9,73 (d, mobiles) -NH-

En plus des produits décrits ci-dessus dans les exemples, les produits suivants constituent des produits pouvant être obtenus selon les méthodes de l'invention :

| $R_1$ | $R_2$ | $R_1$ | $R_2$ |
|---|---|---|---|
| 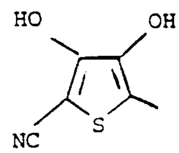 | 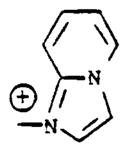 | 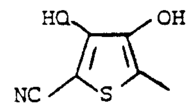 | 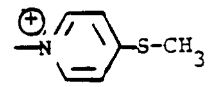 |
| " | 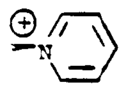 | " | 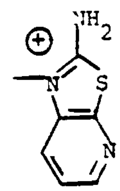 |
| " | 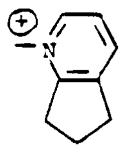 | " | 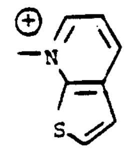 |
| " | 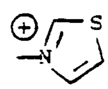 | " | 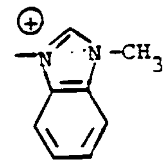 |
| " | 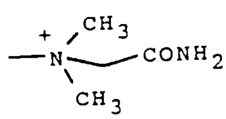 | " | 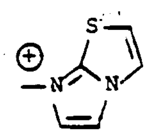 |
| " | 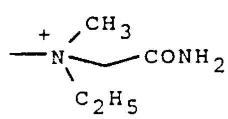 | " | 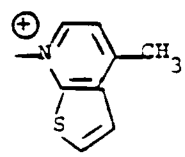 |
| | | " | 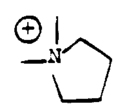 |
| | | " | 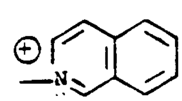 |
| | | " | 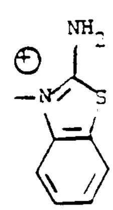 |

| $R_1$ | $R_2$ | $R_1$ | $R_2$ |
|---|---|---|---|
| 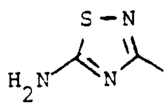 | 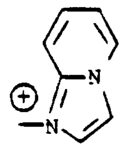 | 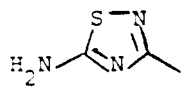 | 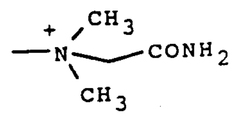 |
| " | 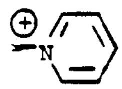 | " | 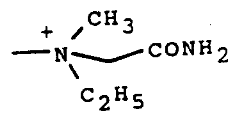 |
| " | 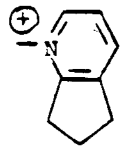 | " | 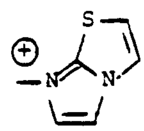 |
| " | 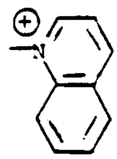 | " | 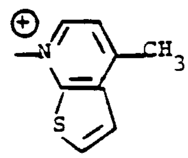 |
| " | 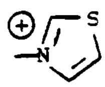 | " | 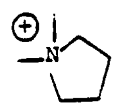 |
| " | 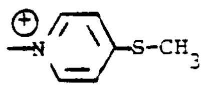 | " | 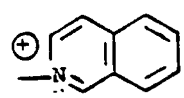 |
| " | 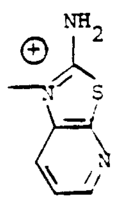 | " | 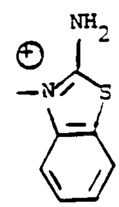 |
| " | 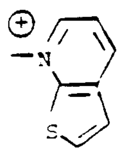 | | |
| " | 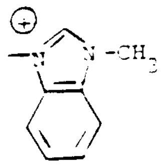 | | |

**EXEMPLE 3 : On a réalisé des préparations pour injections de formule :**

| | |
|---|---|
| - Produit de l'exemple 1 | 500 mg |
| - Excipient aqueux stérile q.s.p. | 5 $cm^3$ |

## ETUDE PHARMACOLOGIOUE

**Activité in vitro, méthode des dilutions en milieu solide.**

On prépare une série de boites dans lesquelles on répartit une même quantité de milieu nutritif stérile, contenant des quantités croissantes du produit à étudier puis chaque boite est ensemencée avec plusieurs souches bactériennes.

Après incubation de 24 heures en étuve à 37°C, l'inhibition de la croissance est appréciée par l'absence de tout développement bactérien ce qui permet de déterminer les concentrations minimales inhibitrices (CMI) exprimées en microgrammes/$cm^3$.

Les résultats sont exprimés en $CMI_{50}$ et $CMI_{90}$ qui est la concentration minimum d'antibiotique permettant d'inhiber la croissance de 50 et 90 % des souches étudiées. On a obtenu les résultats suivants :

| Souches | < > | $CMI_{50}$ | $CMI_{90}$ |
|---|---|---|---|
| Staphylocoques aureus penicilline sensibles (14 souches) | 0,04-0,15 | 0,15 | 0,15 |
| Staphylocoques aureus penicilline résistants (21 souches) | 0,08-1,2 | 0,15 | 0,15 |
| Streptococci groupe D (10 souches) | 2,5-20 | 2,5 | 5 |

| Entérobactéries producteurs de β-lactamases | < > | $CMI_{50}$ | $CMI_{90}$ |
|---|---|---|---|
| Chromosomiques | 0,15-5 | 1,2 | 5 |
| Plasmidiques | | | |
| SHV-2 (13 souches) | 0,08-1,2 | 0,3 | 1,2 |
| SHV-4 (29 souches) | 0,6-20 | 2,5 | 5 |
| TEM-3 (35 souches) | 0,15-20 | 1,2 | 2,5 |

## Revendications

**1.** Les produits de formule générale (I) :

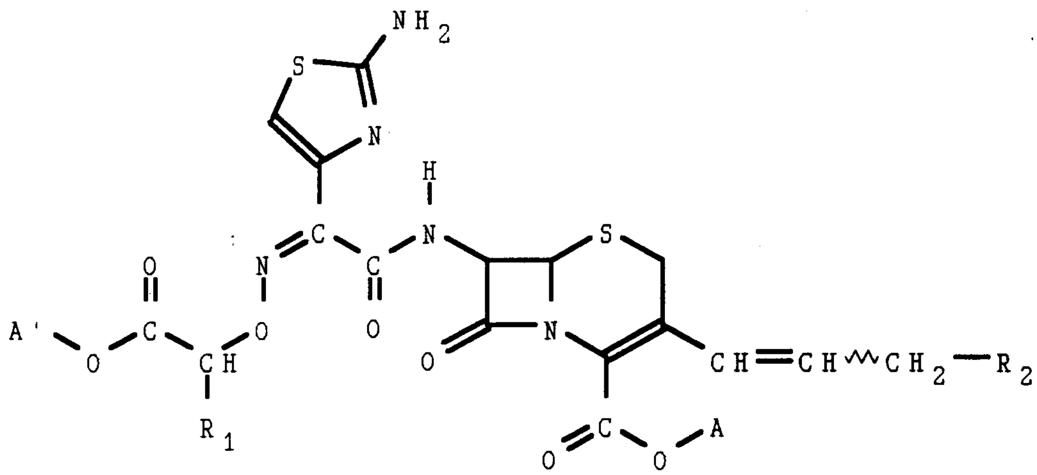

(I)

isomère syn, sous forme (R) ou (S) ou d'un mélange (R,S), sous forme de sel interne ou de leurs sels avec les acides minéraux organiques, formule dans laquelle :
$R_1$ représente un groupement de formule (K) :

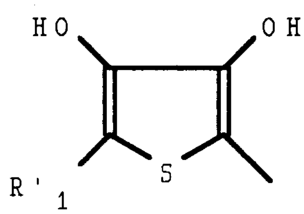

(K)

dans laquelle $R'_1$ représente un radical alkyle renfermant de 1 à 4 atomes de carbone, un radical cyano, carboxy ou alkoxycarbonyle, dans lequel le radical alkyle renferme de 1 à 4 atomes de carbone, ou $R_1$ représente un groupement de formule (L) :

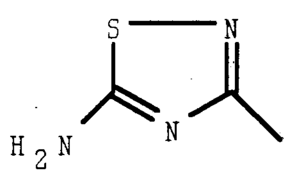

(L)

A et A', identiques ou différents, représentent un atome d'hydrogène, un équivalent de métal alcalin, alcalinoterreux, de magnésium, d'ammonium ou d'une base organique aminée,
ou bien un seul ou chacun des groupes $CO_2A$ ou $CO_2A'$ représentent $CO_2^{\ominus}$,
le trait ondulé signifie que le groupement $CH_2R_2$ peut se trouver dans la position E ou Z et $R_2$ représente sous forme d'ammonium quaternaire, un des radicaux suivants :

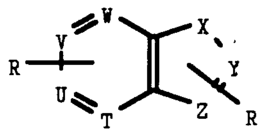
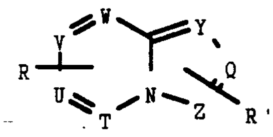
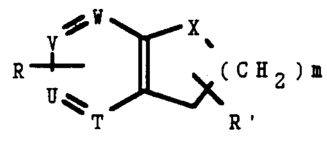
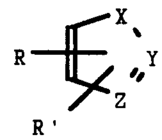

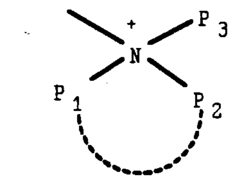

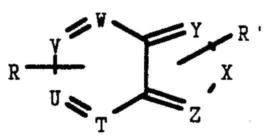
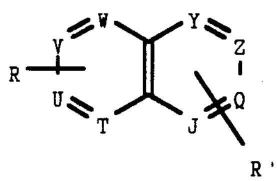
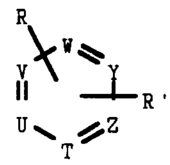
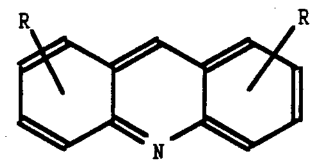

dans lesquels X représente $CH_2$, NH, O ou S ;
Q, J, Y, T, U, V, W et Z, identiques ou différents, représentent indépendamment les uns des autres CH ou N, étant entendu que chacun de ces radicaux cycliques contient de 1 à 5 hétéroatomes, qu'au moins un de ces hétéroatomes est l'atome d'azote et que ces radicaux cycliques sont substitués par un ou plusieurs radicaux R ou R' ;
R et R', identiques ou différents, représentent un atome d'hydrogène, un atome d'halogène, un radical alkyle renfermant de 1 à 4 atomes de carbone, un radical alkyloxy renfermant de 1 à 4 atomes de carbone, un atome d'halogène, un radical cyano, un radical $CO_2$-$Q_1$, CO-$NQ_1(Q_2)$, $NQ_1(Q_2)$, $SO_2$-$NQ_1$-$(Q_2)$, CS-$NH_2$, NH-CO-$Q_1$, CH=N-OH, CH=N-O-$Q_1$, $CH_2$-CN, $CH_2$-S-$Q_1$ dans lesquels $Q_1$ et $Q_2$, identiques ou différents, représentent un atome d'hydrogène ou un radical alkyle renfermant de 1 à 4 atomes de carbone, $P_1$, $P_2$ et $P_3$, identiques ou différents, représentent un radical alkyle renfermant de 1 à 4 atomes de carbone, éventuellement substitué par un des substituants ayant les définitions indiquées ci-dessus pour R et R', le trait pointillé indique que $P_1$ et $P_2$ peuvent éventuellement former avec l'atome d'azote auquel ils sont liés, un hétérocycle à 5 ou 6 chaînons.

**2.** Les produits de formule générale (I) telle que définie à la revendication 1 dans laquelle $R_2$ représente un des radicaux suivants :

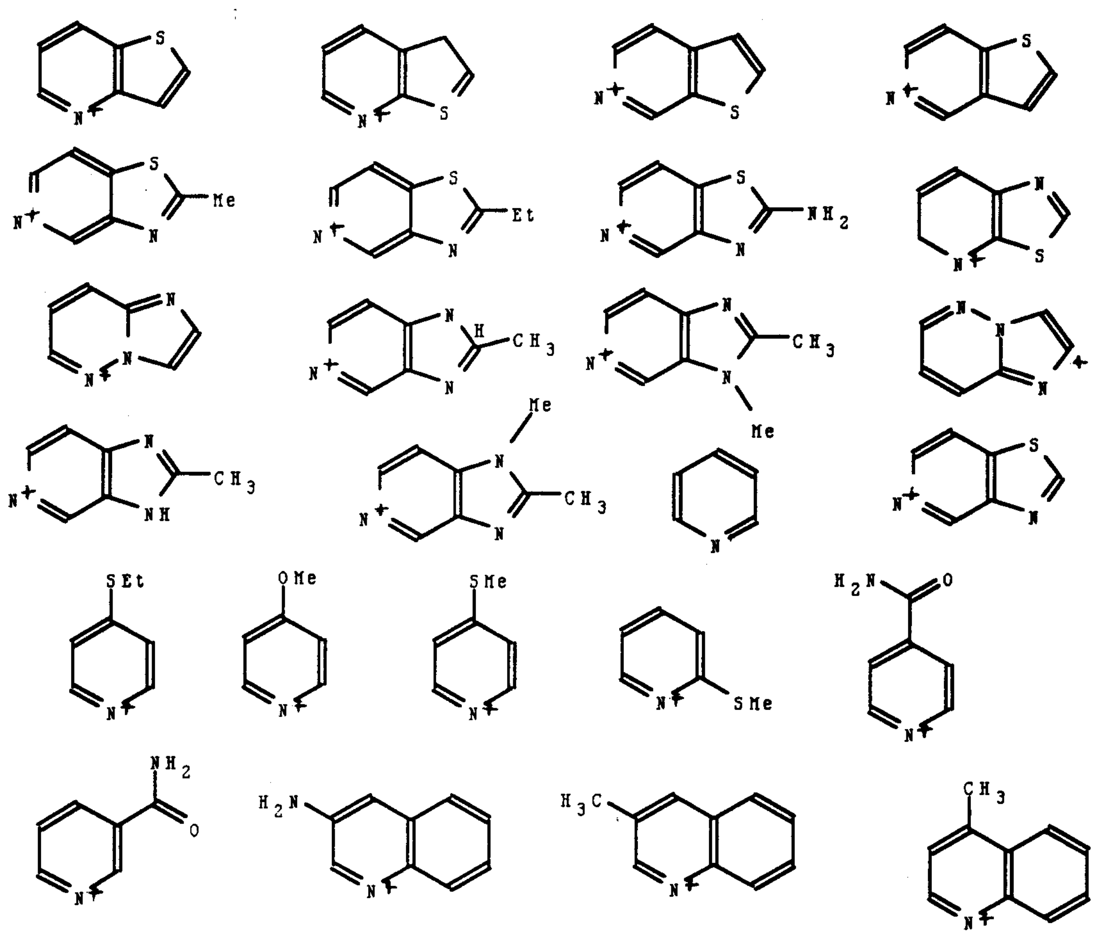

ou encore l'un des radicaux suivants :

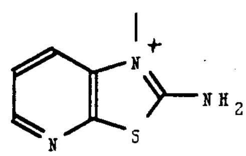

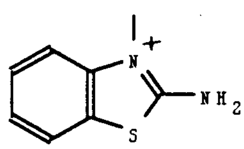

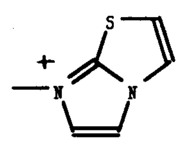

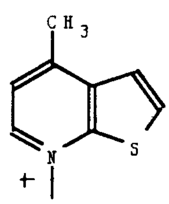

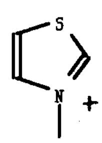

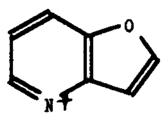 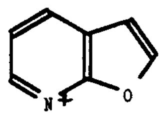 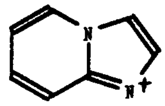 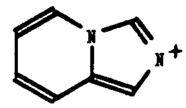

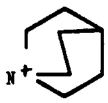 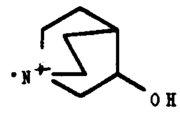 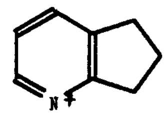 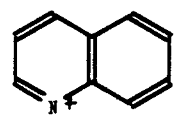

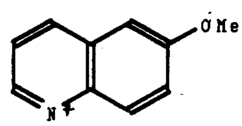 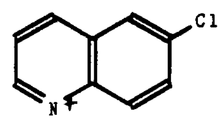 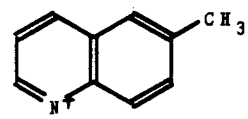

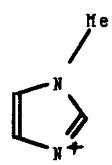 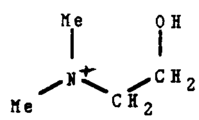 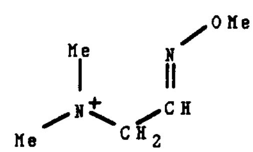 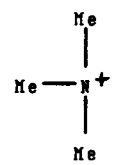

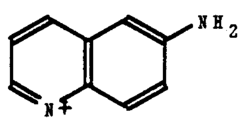 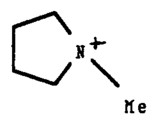 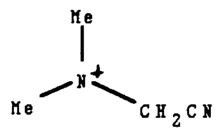 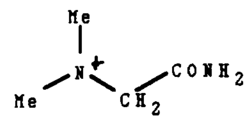

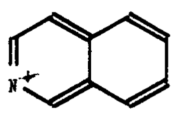 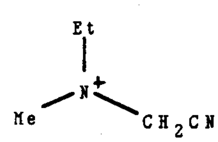 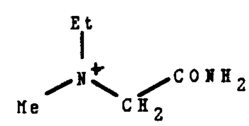

**3.** Les produits de formule générale (I) telle que définie à l'une des revendications 1 et 2, dans laquelle $R_2$ représente le radical quinoléinium, isoquinoléinium, 4-(méthylthio) pyridinium, thiéno[2,3-b]pyridinium, imidazo(1,2-a)pyridinium ou le radical 6,7-dihydro-5H-pyrindinium.

**4.** Les produits de formule générale (I) telle que définie à l'une quelconque des revendications 1, 2 ou 3 dans laquelle $R_1$ représente un radical de formule (K) dans laquelle $R'_1$ représente un radical cyano, carboxy ou alkoxycarbonyle.

**5.** Le sel interne de [6R-[3-(E) 6alpha, 7béta-(Z)]] 1-[3-[7-[[(2-amino 4-thiazolyl) [[carboxy 5-cyano 3,4-dihydroxy 2-thiényl) méthoxy] imino] acétyl] amino] 2-carboxy 8-oxo 5-thia 1-azabicyclo[4,2,0]oct-2-èn-3-yl] 2-propényl] quinoléinium.

**6.** Procédé de préparation des produits de formule (I) telle que définie à la revendication 1, caractérisé en ce que l'on traite un ester de formule (II) :

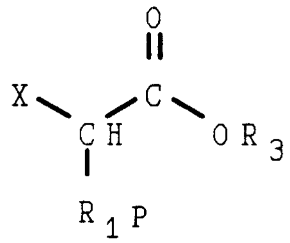

(II)

dans laquelle X représente un groupement partant ou un radical susceptible de générer in situ un groupement partant, $R_1p$ représente un groupement $R_1$ tel que défini plus haut, dans lequel les radicaux hydroxy ou amino sont protégés et $R_3$ représente le reste d'un ester aisément clivable, par le N-hydroxy phtalimide, le cas échéant en présence d'un agent activant, pour conduire au dérivé de formule (III) :

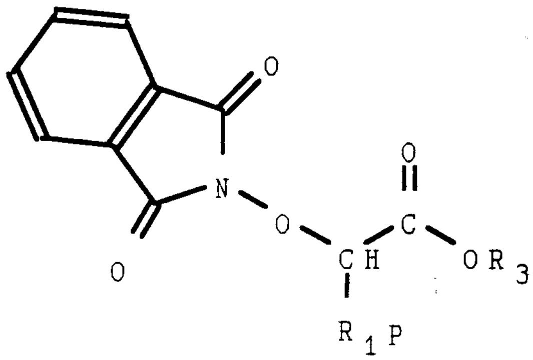

(III)

dérivé de formule (III) que l'on hydrolyse en hydroxylamine O-substituée de formule (IV) :

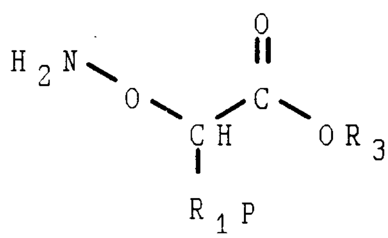

(IV)

produit de formule (IV) que l'on condense avec un dérivé de l'acide (2-amino thiazol-4-yl) 2-oxoacétique de formule (V) :

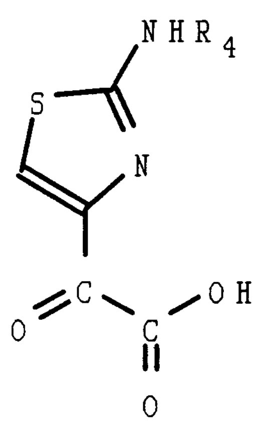

(V)

dans laquelle $R_4$ représente un atome d'hydrogène ou un groupe protecteur de la fonction amine, pour former le dérivé de l'acide alpha-alkoxyimino acétique de formule (VI) :

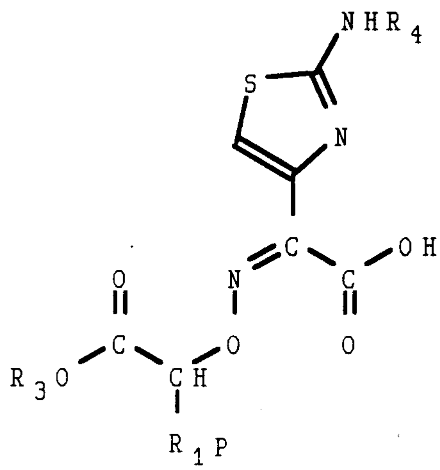

(VI)

dont on prépare, le cas échéant, un dérivé fonctionnel, produit de formule (VI) ou dérivé fonctionnel que l'on amidifie avec un ester du chlorhydrate de l'acide 7-amino 3-(3-halogéno 1-propènyl) 8-oxo 5-thia 1-azabicyclo[4,2,0]oct-2-èn-2-carboxylique de formule (VII) :

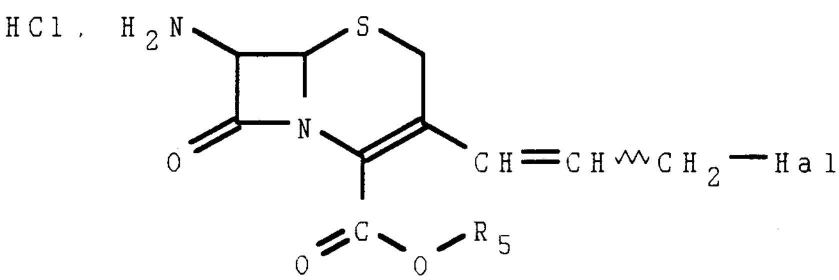

(VII)

ou de ses sels, dans laquelle Hal représente un atome d'halogène et $R_5$ représente le reste d'un ester aisément clivable, pour conduire au dérivé de l'acide 7-(N-substitué amido) 3-(3-halo 1-propènyl) 8-oxo 5-thia 1-azabicyclo[4,2,0]oct-2-èn-2-carboxylique de formule (VIII) :

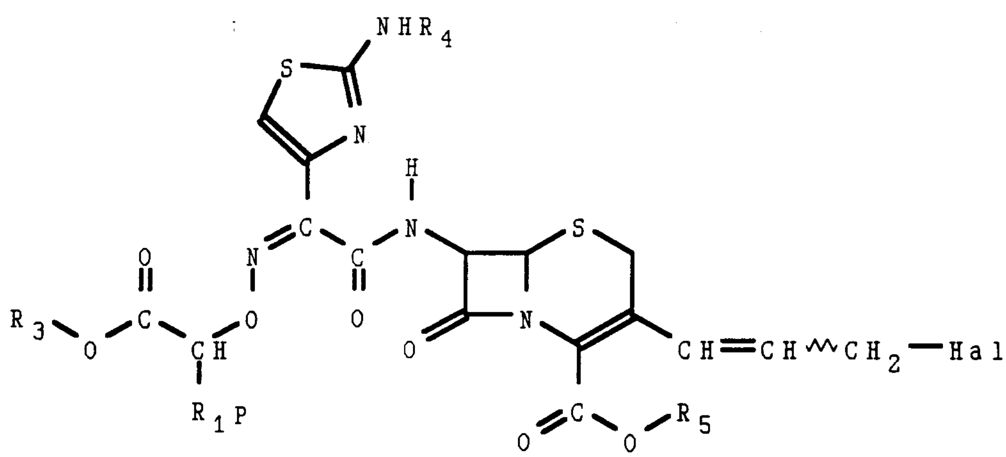

(VIII)

produit de formule (VIII) que l'on transforme, le cas échéant, en analogue 3-(3-iodo propènylé) de formule (IX) :

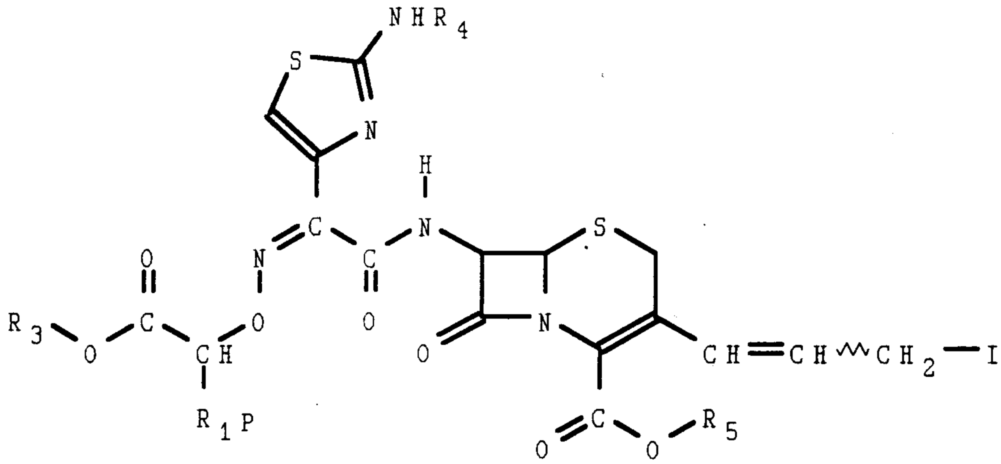

(IX)

produit de formule (IX) que l'on traite avec une base de formule $R_2$ pour obtenir le produit de formule (X) :

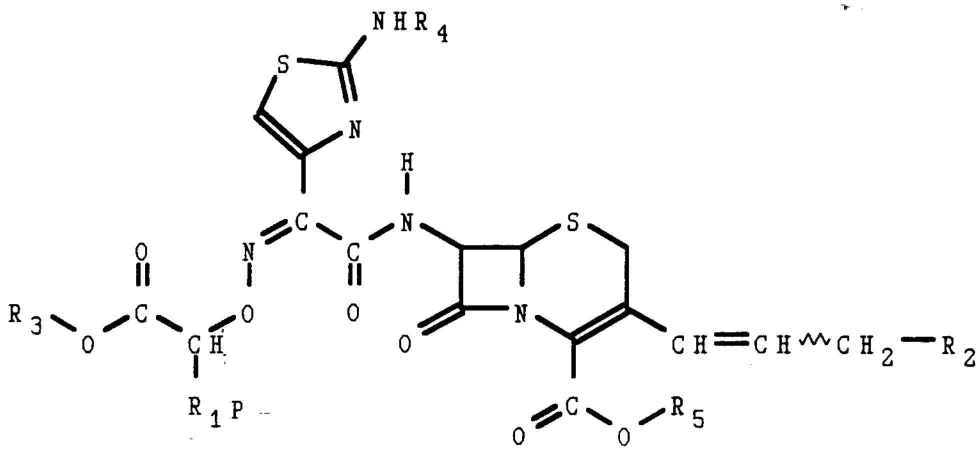

(X)

produit de formule (X) à partir duquel si désiré, l'on isole les isomères (E) ou (Z) ou transforme les isomères (Z) en isomère (E) et produit de formule (X) que l'on soumet à une ou plusieurs des réactions suivantes, dans un ordre quelconque :

a) coupure par hydrolyse ou par action de la thiourée de tout ou partie des groupements esters ou des groupements de protection du ou des radicaux amino ou des radicaux hydroxyles,

b) estérification ou salification par une base du ou des radicaux carboxyliques,

c) salification par un acide du ou des radicaux amino,

d) séparation des produits sous forme de mélange R,S en R ou S.

**7.** Variante du procédé selon la revendication 6, caractérisée en ce que l'hydroxylamine O-substituée de formule (IV) est condensée au produit de formule (XI) :

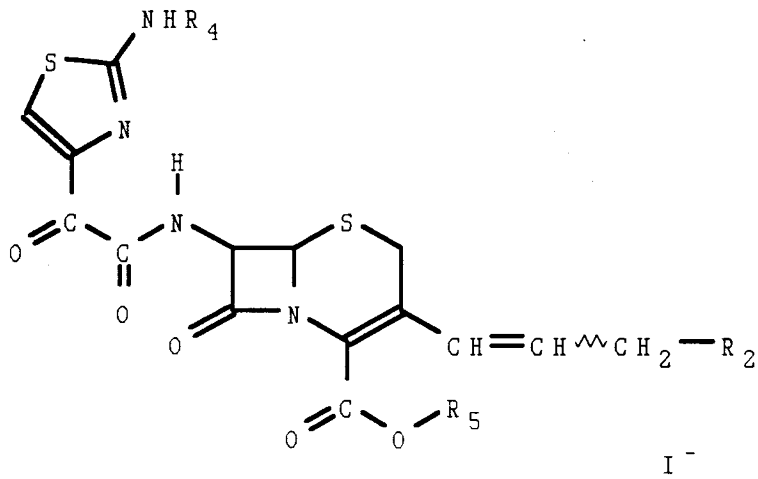

(XI)

pour conduire au produit de formule (X) telle que définie à la revendication 6.

**8.** Procédé selon la revendication 6 ou 7, caractérisé en ce que le composé de formule (II) est préparé par un procédé selon lequel l'on protège, si nécessaire, les fonctions réactives d'un dérivé de formule (XII) :

$R_1$-CHO (XII)

dans laquelle $R_1$ est défini comme à la revendication 1, pour obtenir un dérivé de formule (XIII) :

$R_1$p-CHO (XIII)

dans laquelle $R_1$p est défini comme à la revendication 6, dérivé de formule (XII) ou (XIII) que l'on homologue en dérivé alpha-hydroxy acide de formule (XIV) :

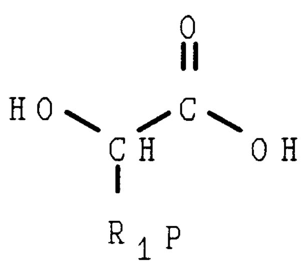

(XIV)

lequel, si désiré, est transformé en dérivé halogéné ou alkyl ou arylsulfonylé correspondant, puis estérifié.

**9.** Procédé selon la revendication 6 ou 7, caractérisé en ce que le composé de formule (II) dans laquelle X représente un atome d'halogène est préparé par un procédé selon lequel l'on traite un aldéhyde de formules (XII) ou (XIII), telles que définies à la revendication 8, par le malononitrile, pour obtenir le dérivé de formule (XV) :

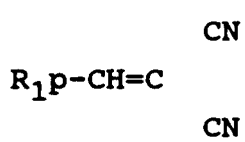

(XV)

que l'on soumet à l'action d'un réactif d'oxydation pour obtenir l'époxyde de formule (XVI) :

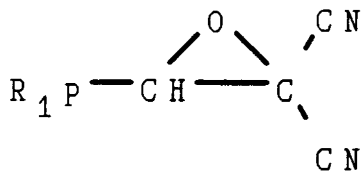

(XVI)

que l'on traite par un acide halohydrique pour obtenir l'acide de formule (XVII) :

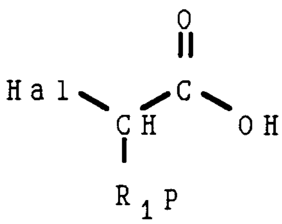

(XVII)

que l'on estérifie.

**10.** A titre de médicaments, les produits répondant à la formule (I) telle que définie à la revendication 1, ainsi que leurs sels d'acides, pharmaceutiquement acceptables.

**11.** A titre de médicaments, les produits tels que définis à l'une quelconque des revendications 2 à 5, ainsi que leurs sels d'acides, pharmaceutiquement acceptables.

**12.** Compositions pharmaceutiques contenant, à titre de principe actif, au moins un médicament selon l'une des revendications 10 ou 11.

**13.** A titre de produits industriels nouveaux, les produits de formules (II), (III), (IV), (VI), (VIII), (IX), et (X), telles que définies à la revendication 6.

**14.** A titre de produits industriels nouveaux, les produits de formules (XIV), (XV), (XVI) et (XVII), telles que définies aux revendications 8 et 9.

Office européen des brevets

# RAPPORT DE RECHERCHE EUROPEENNE

Numero de la demande
EP 93 40 2971

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| Y | EP-A-0 376 724 (TANABE SEIYAKU CO)<br>*page 28-35<br>:examples37,38,42,43,44,45,46,47,48*<br>--- | 1-14 | C07D501/20<br>A61K31/545 |
| Y | EP-A-0 420 608 (YAMANOUCHI PHARMACEUTICAL)<br>*Page 49-51: example 16*<br>* page 108 - page 112; revendications *<br>--- | 1-14 | |
| Y | EP-A-0 315 518 (ROUSSEL-UCLAF)<br>*Document*<br>--- | 1-14 | |
| Y | EP-A-0 462 009 (ROUSSEL-UCLAF)<br>*Document*<br>----- | 1-14 | |
| | | | DOMAINES TECHNIQUES RECHERCHES (Int.Cl.5) |
| | | | C07D |

Le présent rapport a été établi pour toutes les revendications

| Lieu de la recherche | Date d'achèvement de la recherche | Examinateur |
|---|---|---|
| LA HAYE | 10 Février 1994 | LUYTEN H.W. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

## REVENDICATIONS DONNANT LIEU AU PAIEMENT DE TAXES

La présente demande de brevet européen comportait lors de son dépôt plus de dix revendications.

☐ Toutes les taxes de revendication ayant été acquittées dans les délais prescrits, le présent rapport de recherche européenne a été établi pour toutes les revendications.

☐ Une partie seulement des taxes de revendication ayant été acquittée dans les délais prescrits, le présent rapport de recherche européenne a été établi pour les dix premières revendications ainsi que pour celles pour lesquelles les taxes de revendication ont été acquittées,

à savoir les revendications:

☐ Aucune taxe de revendication n'ayant été acquittée dans les délais prescrits, le présent rapport de recherche européenne a été établi pour les dix premières revendications.

## ABSENCE D'UNITE D'INVENTION

La division de la recherche estime que la présente demande de brevet européen ne satisfait pas à l'exigence relative à l'unité d'invention et concerne plusieurs inventions ou pluralités d'inventions,
à savoir:

voir feuille -B-

☐ Toutes les nouvelles taxes de recherche ayant été acquittées dans les délais impartis, le présent rapport de recherche européenne a été établi pour toutes les revendications.

☐ Une partie seulement des nouvelles taxes de recherche ayant été acquittée dans les délais impartis, le présent rapport de recherche européenne a été établi pour les parties de la demande de brevet européen qui se rapportent aux inventions pour lesquelles les taxes de recherche ont été acquittées

à savoir les revendications:

☒ Aucune nouvelle taxe de recherche n'ayant été acquittée dans les délais impartis, le présent rapport de recherche européenne a été établi pour les parties de la demande de brevet européen qui se rapportent à l'invention mentionnée en premier lieu dans les revendications,

à savoir les revendications: 1-12 et 13 (part.)

## ABSENCE D'UNITE D'INVENTION

La division de la recherche estime que la présente demande de brevet européen ne satisfait pas à l'exigence relative à l'unité d'invention et concerne plusieurs inventions ou pluralités d'inventions, à savoir:

1. Revendications 1-12: Céphalosporines, procédé de préparations, compositions pharmaceutiques

   Revendication 13 : Formules VIII, IX,X: Céphalosporines

2. Revendication 13 : Formules II, III, IV,VI: Intermédiaires per se

   Revendication 14 : Intermédiaires per se